(19) Europäisches Patentamt
European Patent Office
Office européen des brevets

(11) EP 0 963 986 B1

(12) **FASCICULE DE BREVET EUROPEEN**

(45) Date de publication et mention de la délivrance du brevet: **17.04.2002 Bulletin 2002/16**

(21) Numéro de dépôt: **99401376.1**

(22) Date de dépôt: **09.06.1999**

(51) Int Cl.[7]: **C07D 471/14**, A61K 31/495, C07D 519/00, C07F 7/08, C07F 7/22 // (C07D471/14, 241:00, 221:00, 221:00)

(54) **Nouveaux dérivés de 5,10-dihydrodipyrido (2,3-b:2,3-e) pyrazine et 5,10-dihydrodipyrido (2,3-b:3,2-e) pyrazine, leur procédé de préparation et les compositions pharmaceutiques qui les contiennent**

5,10-dihydrodipyrido(2,3-b: 2,3-e)pyridazin und 5,10-dihydro(2,3-b:3,2-e)pyrazin, Verfahren zu ihrer Herstellung und diese enhaltende pharmazeutische Zubereitungen

5,10-Dihydrodipyrido(2,3-b:2,3-e)pyrazine and 5,10-dihydro(2,3-b:3,2-e)pyrazine, process for their preparation and pharmaceutical compositions containing them

(84) Etats contractants désignés:
**AT BE CH DE DK ES FI FR GB GR IE IT LI LU NL PT SE**

(30) Priorité: **12.06.1998 FR 9807447**

(43) Date de publication de la demande:
**15.12.1999 Bulletin 1999/50**

(73) Titulaire: **LES LABORATOIRES SERVIER**
**92200 Neuilly sur Seine (FR)**

(72) Inventeurs:
- **Caubere, Paul**
  **64800 Saint-Vincent (FR)**
- **Guillaumet, Gérald**
  **45650 Saint-Jean-le-Blanc (FR)**
- **Rodriguez, Ivan**
  **60290 Cauffry (FR)**
- **Vinter-Pasquier, Karine**
  **88150 Thaon-les-Vosges (FR)**
- **Kuehm-Cuabere, Catherine**
  **92400 Courbevoie (FR)**
- **Blanchard, Stéphanie**
  **45160 Olivet (FR)**
- **Atassi, Ghanem**
  **92210 Saint-Cloud (FR)**
- **Pierre, Alain**
  **78580 Les Alluets-le-Roi (FR)**
- **Pfeiffer, Bruno**
  **95320 Saint-leu-la-Foret (FR)**
- **Renard, Pierre**
  **78150 Le Chesnay (FR)**

(56) Documents cités:
**US-A- 4 593 097**

- **RODRIGUEZ I ET AL: "Design of New Anticancer Drugs. I. Easy Hetarynic Access to Dihydrodipyridopyrazines, a New Family of Antitumor Agents" TETRAHEDRON LETTERS,NL,ELSEVIER SCIENCE PUBLISHERS, AMSTERDAM, vol. 39, no. 40, 1 octobre 1998 (1998-10-01), pages 7283-7286, XP004133659 ISSN: 0040-4039**

Remarques:
Le dossier contient des informations techniques présentées postérieurement au dépôt de la demande et ne figurant pas dans le présent fascicule.

EP 0 963 986 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne de nouveaux dérivés de 5,10-dihydrodipyrido [2,3-*b* : 2,3-*e*] et [2,3-*b* : 3,2-*e*] pyrazine, leur procédé de préparation et les compositions pharmaceutiques qui les contiennent.

Le brevet US 4,953,097 décrit des dérivés de phénazine et les revendique pour leurs activités antibactériennes et anti-tumorales.

Les composés de l'invention, outre le fait qu'ils soient nouveaux, présentent de façon surprenante une activité in-vitro et in-vivo intéressante pour ce type de composés. Les nouveaux composés, découverts par la Demanderesse, possèdent ainsi des propriétés antitumorales qui les rendent particulièrement adaptés pour une utilisation dans le traitement des cancers.

Plus spécifiquement, la présente invention concerne les composés de formule **(I)** :

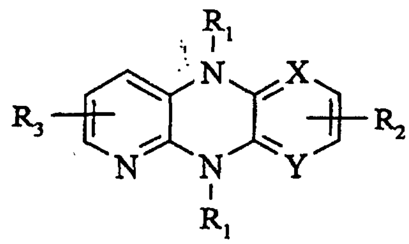

**(I)**

dans laquelle :

**X** représente un atome d'azote, de carbone, ou un groupement CH,

**Y** représente un atome d'azote quand X représente un atome de carbone ou un groupement CH, ou Y représente un atome de carbone, ou un groupement CH, quand X représente un atome d'azote,

étant entendu que lorsque X et Y représente un atome de carbone alors celui-ci porte le groupement $R_2$,

**$R_1$** représente un groupement alkyle ($C_1$-$C_6$) linéaire ou ramifié (éventuellement substitué par un ou plusieurs groupements, identiques ou différents, choisis parmi halogène, alkylthio ($C_1$-$C_6$) linéaire ou ramifié, alkoxy ($C_1$-$C_6$) linéaire ou ramifié, monoalkyle ou dialkylamino ($C_1$-$C_6$) linéaire ou ramifié, aryle, hydroxy, formyle, alkyloxycarbonyle ($C_1$-$C_6$) linéaire ou ramifié, et carboxy),
étant entendu que les deux groupements $R_1$ ont soit des définitions de groupements identiques, soit des définitions de groupements différentes,

**$R_2$ et $R_3$,** identiques ou différents, indépendamment l'un de l'autre, représentent un groupement Z,
<u>ou</u>
l'un desdits groupements $R_2$ ou $R_3$ représente un groupement W et l'autre desdits groupements $R_2$ ou $R_3$ représente un groupement Z,
groupements Z et W dans lesquels :

**-**Z représente :

* un atome d'hydrogène,
* d'halogène,
* un groupement alkyle ($C_1$-$C_6$) linéaire ou ramifié, ledit groupement alkyle pouvant être substitué par un ou plusieurs groupements, identiques ou différents, choisis parmi hydroxy, alkoxy ($C_1$-$C_6$) linéaire ou ramifié, aryle, amino (éventuellement substitué par un ou deux groupements, identiques ou différents, choisis parmi alkyle ($C_1$-$C_6$) linéaire ou ramifié, aryle, arylalkyle ($C_1$-$C_6$) linéaire ou ramifié, aminoalkyle ($C_1$-$C_6$) linéaire ou ramifié, monoalkylaminoalkyle ($C_1$-$C_6$) linéaire ou ramifié, dialkylaminoalkyle ($C_1$-$C_6$) linéaire ou ramifié, hydroxyalkyle ($C_1$-$C_6$) linéaire ou ramifié, et alkoxyalkyle ($C_1$-$C_6$) linéaire ou ramifié), et formyle,
* amino (éventuellement substitué par un ou deux groupements, identiques ou différents, choisis parmi alkyle ($C_1$-$C_6$) linéaire ou ramifié, aryle, arylalkyle ($C_1$-$C_6$) linéaire ou ramifié, alkylsulfonyle ($C_1$-$C_6$) linéaire ou ramifié, arylsulfonyle, aminoalklyle ($C_1$-$C_6$) linéaire ou ramifié, monoalkylaminoalkyle ($C_1$-$C_6$) linéaire ou ramifié, dialkylaminoalkyle ($C_1$-$C_6$) linéaire ou ramifié, hydroxyalkyle

($C_1$-$C_6$) linéaire ou ramifié, et alkoxyalkyle ($C_1$-$C_6$) linéaire ou ramifié),
- * nitro,
- * alkylthio ($C_1$-$C_6$) linéaire ou ramifié,
- * formyle,
- * hydroxycarbonyle,
- * alkoxycarbonyle ($C_1$-$C_6$) linéaire ou ramifié,
- * aminocarbonyle, monoalkylaminocarbonyle ($C_1$-$C_6$) linéaire ou ramifié, ou un groupement dialkylaminocarbonyle ($C_1$-$C_6$) linéaire ou ramifié,

**-**W représente une chaîne alkylène ($C_5$-$C_{24}$) linéaire ou ramifiée, dans laquelle un ou plusieurs atomes de carbone sont éventuellement remplacés par un ou plusieurs groupements, identiques ou différents, indépendamment l'un de l'autre, choisis parmi atomes d'oxygène, groupements imine, -N($R_4$)-CO-, -CO-N($R_4$)-, et N($R_4$) dans lesquels $R_4$ représente un atome d'hydrogène, un groupement alkyle ($C_1$-$C_6$) linéaire ou ramifié, aryle, ou arylalkyle ($C_1$-$C_6$) linéaire ou ramifié, ladite chaîne alkylène étant substituée dans sa position terminale par l'un quelconque des groupements suivants :

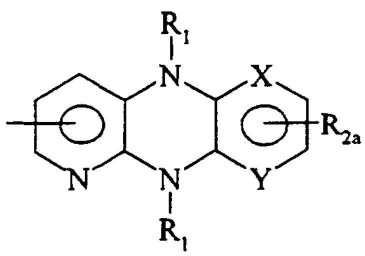
,
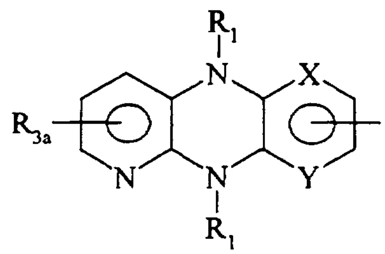

dans lesquels X, Y et $R_1$ sont tels que définis précédemment, et $R_{2a}$ et $R_{3a}$ représentent un groupement Z tel que défini précédemment,

leurs isomères ainsi que leurs sels d'addition à un acide ou à une base pharmaceutiquement acceptable.

Parmi les acides pharmaceutiquement acceptables, on peut citer à titre non limitatif les acides chlrohydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Par groupement aryle, on entend un groupement phényle, naphtyle, tétrahydronapthyle, dihydronaphtyle, indényle ou indanyle, chacun de ces groupements étant éventuellement substitué de façon identique ou différente, par un ou plusieurs atomes d'halogène, groupements hydroxy, cyano, nitro, alkyle ($C_1$-$C_6$) linéaire ou ramifié, trihalogénoalkyle ($C_1$-$C_6$) linéaire ou ramifié, alkoxy ($C_1$-$C_6$) linéaire ou ramifié, acyle ($C_1$-$C_6$) linéaire ou ramifié, carboxy, alkoxycarbonyle ($C_1$-$C_6$) linéaire ou ramifié, ou amino (substitué éventuellement par un ou deux groupements, identiques ou différents, choisis parmi alkyle ($C_1$-$C_6$) linéaire ou ramifié, aryle et arylakyle ($C_1$-$C_6$) linéaire ou ramifié).

Selon une variante avantageuse de l'invention, les composés préférés sont ceux pour lesquels $R_2$ et $R_3$, identiques ou différents, indépendamment l'un de l'autre, représentent un groupement Z.

Selon une autre variante avantageuse, les composés préférés de l'invention sont ceux pour lesquels l'un des groupements $R_2$ ou $R_3$ représente un groupement Z et l'autre des groupements $R_2$ ou $R_3$ représente un groupement W.

Selon une variante particulièrement préférentielle, les composés préférés de l'invention sont ceux pour lesquels l'un des groupements $R_2$ ou $R_3$ représente un groupement Z et l'autre des groupements $R_2$ ou $R_3$ représente un groupement $W_1$, ledit groupement $W_1$ représentant une chaîne alkylène ($C_6$-$C_{12}$) linéaire dans laquelle deux ou trois atomes de carbone sont remplacés par deux ou trois groupements, identiques ou différents, N($R_4$) dans lesquels $R_4$, identiques ou différents, représente un atome d'hydrogène ou un groupement alkyle ($C_1$-$C_6$) linéaire ou ramifié, ladite chaîne alkylène étant substituée dans sa position terminale par l'un quelconque des deux groupements suivants :

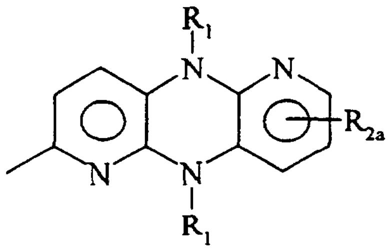

,

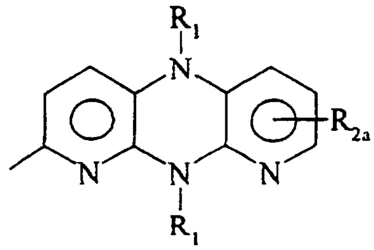

dans lesquels $R_1$ est tel que défini dans la formule générale (I) et $R_{2a}$ représente un groupement Z.

Selon une autre variante préférentielle, les composés préférés de l'invention sont ceux pour lesquels l'un des groupements $R_2$ ou $R_3$ représente un groupement Z et l'autre des groupements $R_2$ ou $R_3$ représente un groupement $W_2$ de formule :

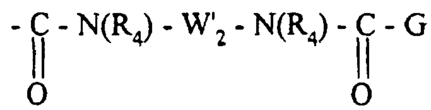

dans laquelle

- ♦ $R_4$, identique ou différent, représente un atome d'hydrogène ou un groupement alkyle ($C_1$-$C_6$) linéaire ou ramifié,
- ♦ $W'_2$ représente une chaîne alkylène ($C_3$-$C_{12}$) linéaire, dans laquelle, un, deux ou trois atomes de carbone sont éventuellement remplacés par un, deux ou trois groupements, identiques ou différents, $N(R_4)$ dans lesquels $R_4$ est tel que défini précédemment,
- ♦ G représente l'un quelconque des deux groupements suivants :

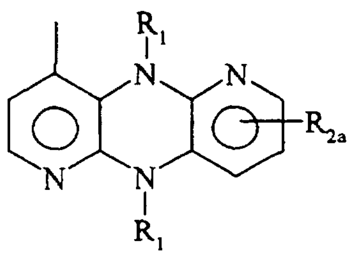

,

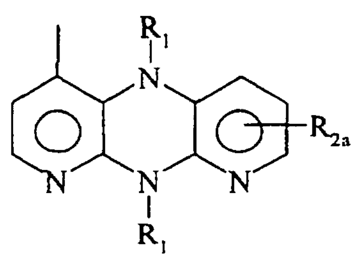

dans lesquels $R_1$ est tel que défini dans la formule générale (I) et $R_{2a}$ représente un groupement Z.

D'une façon préférentielle, les composés préférés de l'invention sont ceux pour lesquels l'un des groupements $R_2$ ou $R_3$ représente un groupement $W_1$ ou $W_2$ tel que défini précédemment, dans lesquels le groupement $R_1$ représente un groupement alkyle ($C_1$-$C_6$) linéaire ou ramifié, et $R_{2a}$ représente un atome d'hydrogène, et l'autre des groupements $R_2$ ou $R_3$ représente un groupement Z correspondant à un atome d'hydrogène.

Le substituant $R_1$ préféré selon l'invention est le groupement alkyle ($C_1$-$C_6$) linéaire ou ramifié.

D'une façon particulièrement intéressante, les composés préférés de l'invention sont :

- le N1,N2-di[(5,10-diméthyl-5,10-dihydrodipyrido-[2,3-b:3,2-e]pyrazin-2-yl)méthyl]-1,2-éthanediamine,
- le N1,N3-di[(5,10-diméthyl-5,10-dihydrodipyrido-[2,3-b:3,2-e]pyrazin-2-yl)méthyl]-1,3-propanediamine,
- le N1,N4-di[(5,10-diméthyl)-5,10-dihydrodipyrido-[2,3-b:3,2-e]pyrazin-2-yl)méthyl]-1,4-butanediamine,
- le N1-[(5,10-diméthyl-5,10-dihydrodipyrido-[2,3-b:3,2-e]pyrazin-2-yl)méthyl]-N3-(3-{[5,10-diméthyl-5,10-dihydrodipyrido[2,3-b:3,2-e]pyrazin-2-yl)méthyl]amino}propyl)-N3-méthyl-1,3-propanediamine,
- et le N1,N7-di[(5,10-diméhtyl-5,10-dihydrodipyrido-[2,3-b:3,2-e]pyrazin-2-yl)méthyl]-1,7-heptanediamine.

Les isomères ainsi que les sels d'addition à un acide ou à une base pharmaceutiquement acceptable, des composés préférés, font partie intégrante de l'invention.

L'invention s'étend également au procédé de préparation des composés de formule (I), caractérisé en ce que

l'on utilise comme produit de départ une 3-halogéno-2-amino-pyridine de formule **(II)** :

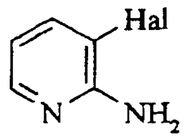

**(II)**

dans laquelle Hal représente un atome d'halogène, composés de formule (II) que l'on fait réagir en condition basique avec un composé de formule **(III)** :

$R'_1$ - Hal **(III)**

dans laquelle Hal représente un atome d'halogène et $R'_1$ représente un groupement alkyle ($C_1$-$C_6$) linéaire ou ramifié, éventuellement substitué par un groupement alkoxy ($C_1$-$C_6$) linéaire ou ramifié, monoalkyle ou dialkylamino ($C_1$-$C_6$) linéaire ou ramifié, ou un groupement aryle,
pour conduire aux composés de formule **(IV)** :

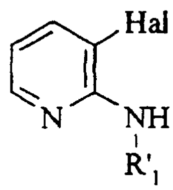

**(IV)**

dans laquelle Hal et $R'_1$ sont tels que définis précédemment, composé de formule (IV) qui est condensé sur lui-même en présence d'une base complexe, pour conduire aux composés de formule **(I/a)**, cas particulier des composés de formule (I) :

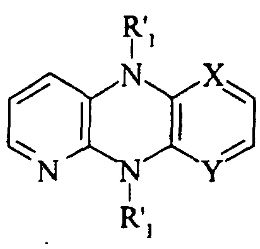

**(I/a)**

dans laquelle $R'_1$ est tel que défini précédemment et, X et Y ont la même signification que dans la formule (I), composés de formule (I/a), dans le cas particulier où $R'_1$ représente un groupement $R''_1$ consistant en un groupement alkyle ($C_1$-$C_6$) linéaire, que l'on soumet si on le désire à une réaction de métallation et dont on piège l'anion intermédiaire par un halogénure d'alkyle ($C_1$-$C_6$) dialkyldisulfure ($C_1$-$C_6$) linéaire ou ramifié, un halogénure d'alkyl ($C_1$-$C_6$) alkoxy ($C_1$-$C_6$) linéaire ou ramifié, un halogène, un aldéhyde, une cétone, un dérivé carboxylé ou alkoxycarbonylé ($C_1$-$C_6$) linéaire ou ramifié, pour conduire aux composés de formules **(I/b)** et **(I/b')**, et/ou **(I/c)**, les produits (I/b') étant des coproduits de réactions :

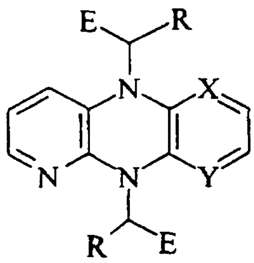 (et/ou) 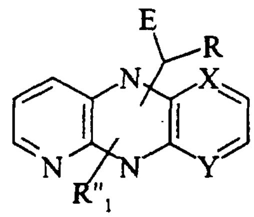 (et/ou) 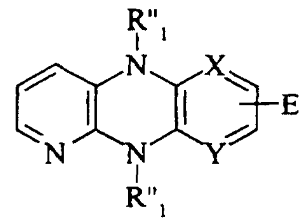

**(I/b)** **(I/b')** **(I/c)**

dans lesquelles X, Y et R"$_1$ sont tels que définis précédemment, R représente un atome d'hydrogène ou un groupement alkyle ($C_1$-$C_5$) linéaire et E représente un atome d'halogène, un groupement alkyle ($C_1$-$C_6$) linéaire ou ramifié, alkylthio ($C_1$-$C_6$) linéaire ou ramifié, formyle, alkyle ($C_1$-$C_6$) linéaire ou ramifié substitué par un groupement alkoxy ($C_1$-$C_6$) linéaire ou ramifié, ou alkyle ($C_1$-$C_6$) linéaire ou ramifié substitué par un ou plusieurs groupements, identiques ou différents, choisis parmi aryle, hydroxy, formyle, alkoxycarbonyle ($C_1$-$C_6$) linéaire ou ramifié, et carboxy,
l'ensemble des composés de formule (I/a), (I/b) et (I/b') formant les composés de formule **(I/d)** :

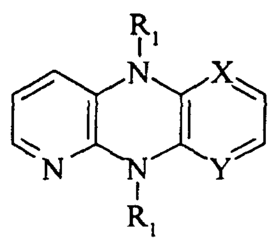

**(I/d)**

dans laquelle $R_1$, X et Y ont la même signification que dans la formule (I),
composés de formule (I/d),

❖ que l'on traite si on le désire par un agent de nitration, pour conduire aux composés de formule **(I/e)**, cas particulier des composés de formule **(I)**,

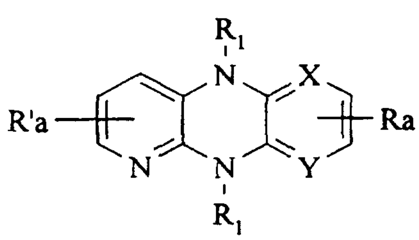

**(I/e)**

dans laquelle X, Y et $R_1$ sont tels que définis précédemment,
et Ra et R'a, identiques ou différents, représentent un atome d'hydrogène ou un groupement nitro, étant entendu que Ra et R'a ne peuvent pas représenter simultanément un atome d'hydrogène,
composés de formule (I/e) dont on réduit si on le souhaite la (les) fonction(s) nitro en fonction amine, pour conduire aux composés de formule **(I/f)**, cas particulier des composés de formule (I) :

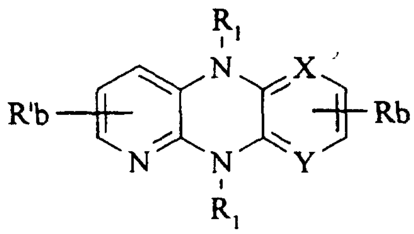

**(I/f)**

dans laquelle X, Y et $R_1$ sont tels que définis précédemment et Rb, R'b, identiques ou différents, représentent un atome d'hydrogène ou un groupement amino, étant entendu que Rb et R'b ne peuvent pas représenter simultanément un atome d'hydrogène,
composés de formule (I/f) que l'on traite en condition basique avec un dérivé électrophile, pour conduire aux composés de formule **(I/g)**, cas particulier des composés de formule (I) :

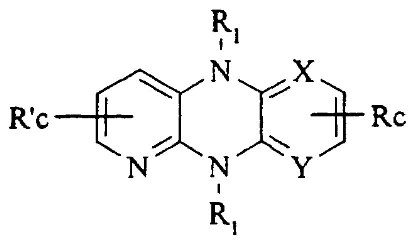

**(I/g)**

dans laquelle X, Y et $R_1$ sont tels que définis précédemment, et Rc, R'c, identiques ou différents, représentent un atome d'hydrogène ou un groupement amino substitué par un ou deux groupements, identiques ou différents, choisis parmi alkyle ($C_1$-$C_6$) linéaire ou ramifié, aryle, arylalkyle ($C_1$-$C_6$) linéaire ou ramifié, alkylsulfonyle ($C_1$-$C_6$) linéaire ou ramifié, arylsulfonyle, aminoalkyle ($C_1$-$C_6$) linéaire ou ramifié, monoalkylaminoalkyle ($C_1$-$C_6$) linéaire ou ramifié, dialkylaminoalkyle ($C_1$-$C_6$) linéaire ou ramifié, hydroxyalkyle ($C_1$-$C_6$) linéaire ou ramifié, et alkoxyalkyle ($C_1$-$C_6$) linéaire ou ramifié, étant entendu que Rc et R'c ne peuvent pas représenter simultanément un atome d'hydrogène,

❖ ou que l'on soumet à des conditions de réaction de formylation, pour conduire aux composés de formule **(I/h)**, cas particulier des composés de formule (I) .

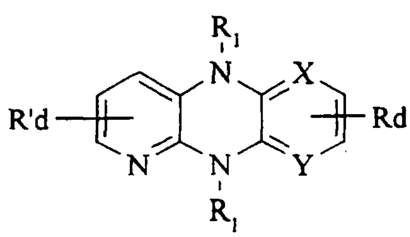

**(I/h)**

dans laquelle X, Y et $R_1$ sont tels que définis précédemment et Rd, R'd, identiques ou différents, représentent un groupement formyle ou un atome d'hydrogène, étant entendu que Rd et R'd ne peuvent pas représenter simultanément un atome d'hydrogène,
composés de formule (I/h)

- que l'on traite par un agent oxydant puis que l'on soumet éventuellement à l'action d'un alcool, pour conduire aux composés de formule **(I/h')**, cas particulier des composés de formule (I)

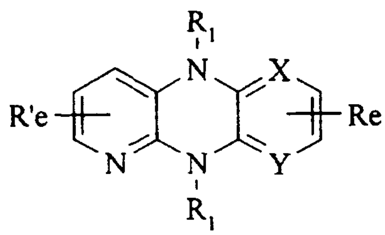

(I/h')

dans laquelle $R_1$, X et Y sont tels que définis précédemment et Re, R'e, identiques ou différents, représentent un atome d'hydrogène, un groupement hydroxycarbonyle ou un groupement alkoxycarbonyle ($C_1$-$C_6$) linéaire ou ramifié, étant entendu que R'e et Re ne peuvent pas représenter simultanément un atome d'hydrogène,
composés de formule (I/h') dont on transforme éventuellement au moins l'un des groupements Re et/ou R'e en chlorure d'acide, selon des conditions classiques de synthèse organique, ou que l'on fait réagir directement avec un composé amino, selon par exemple des conditions de couplage peptidique, pour conduire aux composés de formule (I/h'$_1$), cas particulier des composés de formule (I) :

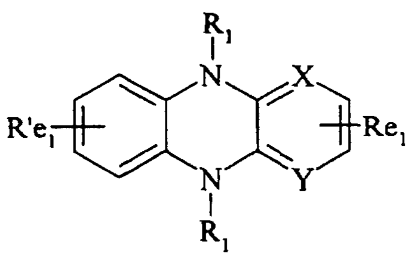

(I/h'$_1$)

dans laquelle $R_1$, X et Y sont tels que définis précédemment et $Re_1$, $R'e_1$, identiques ou différents, représentant un atome d'hydrogène, un groupement aminocarbonyle, monoalkylaminocarbonyle ($C_1$-$C_6$) linéaire ou ramifié, ou dialkylaminocarbonyle ($C_1$-$C_6$) linéaire ou ramifié, étant entendu que $Re_1$ et $R'e_1$ ne peuvent pas représenter simultanément un atome d'hydrogène,

- ou que l'on soumet à des conditions d'amination réductrice pour conduire aux composés de formule **(I/i)**, cas particulier des composés de formule (I) :

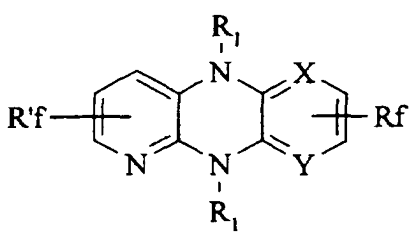

**(I/i)**

dans laquelle X, Y et $R_1$ sont tels que définis précédemment et Rf, R'f, identiques ou différents, représentent un atome d'hydrogène ou un groupement de formule $CH_2$-$NR_{10}$-$R_{20}$ dans lequel $R_{10}$ et $R_{20}$, identiques ou différents, représentent un atome d'hydrogène, un groupement alkyl ($C_1$-$C_6$) linéaire ou ramifié, aryle, arylalkyle ($C_1$-$C_6$) linéaire ou ramifié, aminoalkyle ($C_1$-$C_6$) linéaire ou ramifié, monoalkylaminoalkyle ($C_1$-$C_6$) linéaire ou ramifié, dialkylaminoalkyle ($C_1$-$C_6$) linéaire ou ramifié, hydroxyalkyle ($C_1$-$C_6$) linéaire ou ramifié, ou alkoxyalkyle ($C_1$-$C_6$) linéaire ou ramifié, étant entendu que Rf et R'f ne peuvent pas représenter simultanément un atome d'hydrogène,

- ou que l'on traite par un ylure de phosphore préparé à partir d'un composé de formule **(V)**.

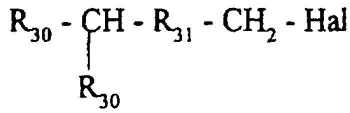

**(V)**

dans laquelle Hal représente un atome d'halogène, $R_{31}$ représente une chaîne alkylène ($C_1$-$C_4$) linéaire ou ramifiée, et $R_{30}$ représente un groupement protecteur des fonctions aldéhydes, tel qu'un acétal, pour conduire aux composés de formule **(VI)** :

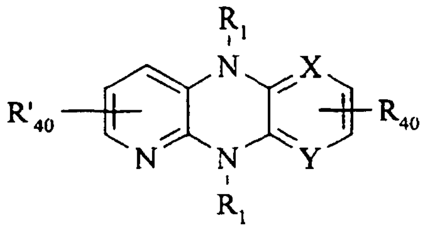

**(VI)**

dans laquelle X, Y et $R_1$ sont tels que définis précédemment et $R_{40}$ et $R'_{40}$ représentent un atome d'hydrogène ou un groupement de formule :

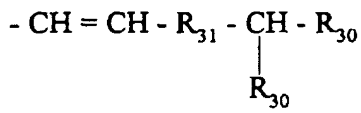

dans laquelle $R_{31}$ et $R_{30}$ sont tels que définis précédemment, composés de formule (VI) dont on réduit la double liaison selon des conditions classiques de synthèse organique, puis dont on déprotège la fonction aldéhyde terminale, pour conduire aux composés de formule **(I/j)**, cas particulier des composés de formule (I) :

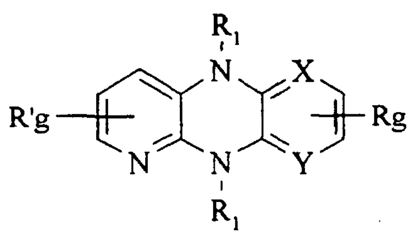

**(I/j)**

dans laquelle X, Y et $R_1$ sont tels que définis précédemment et, Rg, R'g, identiques ou différents, représentent un atome d'hydrogène ou un groupement alkyle ($C_1$-$C_6$) linéaire ou ramifiée, substitué par un groupement formyle, étant entendu que Rg et R'g ne peuvent pas représenter simultanément un atome d'hydrogène, composés de formule (I/j) que l'on soumet à des conditions d'amination réductrice pour conduire aux composés de formule (**I/k**), cas particulier des composés de formule (I) :

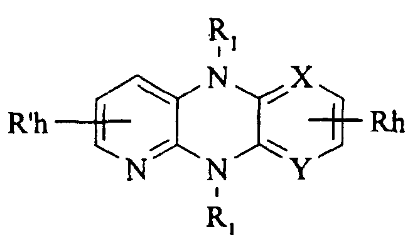

**(I/k)**

dans laquelle X, Y et $R_1$ sont tels que définis précédemment et Rh, R'h, identiques ou différents, représentent un atome d'hydrogène ou un groupement alkyle ($C_1$-$C_6$) linéaire ou ramifié, substitué par un groupement de

formule $CH_2$-$NR_{10}R_{20}$ dans lequel $R_{10}$ et $R_{20}$ sont tels que définis précédemment, étant entendu que Rh et R'h ne peuvent pas représenter simultanément un atome d'hydrogène,

❖ ou que l'on soumet à un agent d'halogénation, tel que par exemple le tribromure de phényltriméthylammonium, pour conduire aux composés de formule **(I/l)**, cas particulier des composés de formule (I) :

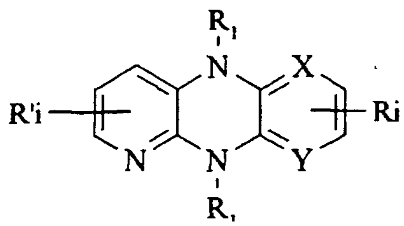

(I/l)

dans laquelle X, Y et $R_1$ sont tels que définis précédemment, et Ri, R'i, identiques ou différents représentent un atome d'hydrogène ou un atome d'halogène, étant entendu que Ri et R'i ne représentent pas simultanément un atome d'hydrogène,

étant entendu que les réactions précédemment décrites, effectuées à partir des composés de formule (I/d), permettent d'obtenir un ou plusieurs produits substitués soit sur un cycle pyridinyle, soit sur l'autre cycle pyridinyle soit sur les deux cycles pyridinyle, que ces différentes réactions peuvent être réalisées dans un ordre quelconque à partir des composés de formule (I/d) et/ou (I/c) et qu'ainsi selon un choix judicieux de l'homme de l'art, tant au niveau des réactifs utilisés, de l'ordre dans lequel les réactions sont effectuées, que des conditions de réaction, il est possible d'obtenir à partir des composés de formule (I/d) et (I/c) l'un quelconque des composés de formule **(I/m)**, cas particulier des composés de formule (I) :

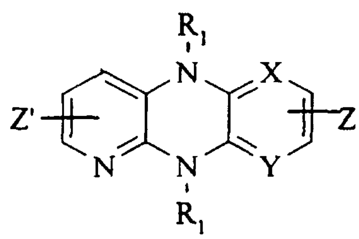

(I/m)

dans laquelle X, Y, $R_1$ et Z sont tels que définis dans la formule (I), et Z' représente l'une quelconque des définitions de Z,

*composés de formule (I/m)*, que l'on traite, si on le désire,

⇨ soit, dans le cas où l'un des groupements Z ou Z' représente un groupement amino ou aminoalkyle ($C_1$-$C_7$) linéaire ou ramifié, dans des conditions d'animation ou d'amination réductrice,

* *par un composé* de formule **(VII)** :

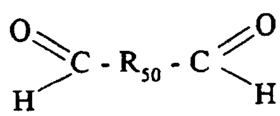

(VII)

dans laquelle $R_{50}$ représente une chaîne alkylène linéaire ou ramifiée, dans laquelle un ou plusieurs atomes de carbone sont remplacés éventuellement par un ou plusieurs atomes d'oxygène ou groupements $N(R_4)$ avec $R_4$ tel que défini dans la formule (I),

* ou *par un composé* de formule (**VIII**) :

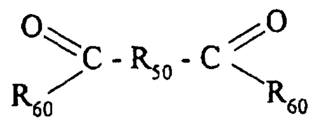

**(VIII)**

dans laquelle $R_{60}$ représente un atome de chlore ou un groupement alkoxy ($C_1$-$C_6$) linéaire ou ramifié, et $R_{50}$ est tel que défini précédemment,

⇨ soit, dans le cas où l'un des groupements Z ou Z' représente un groupement formyle ou formylalkyle ($C_1$-$C_6$) linéaire ou ramifié, dans des conditions d'amination ou d'amination réductrice,
par un composé de formule **(IX)** :

$$HR_4N - R_{70} - NR_4H \qquad \textbf{(IX)}$$

dans laquelle $R_4$ est tel que défini dans la formule (I) et $R_{70}$ représente une chaîne alkylène linéaire ou ramifié, dans laquelle un ou plusieurs atomes de carbone peuvent éventuellement être remplacés par un ou plusieurs atomes d'oxygène ou groupement $NR_4$ avec $R_4$ tel que défini précédemment,

⇨ soit, dans le cas où l'un des groupements Z ou Z' représente un groupement alkoxycarbonyle ($C_1$-$C_6$) linéaire ou ramifié, ou un chlorure d'acide, par un composé de formule (IX) tel que défini précédemment, dans des conditions classiques de couplage peptidique,

pour conduire aux composés de formule **(I/n)** ou **(I/o)**, cas particulier des composés de formule (I) :

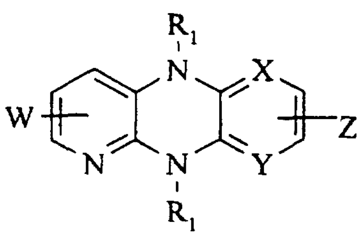

**(I/n)**

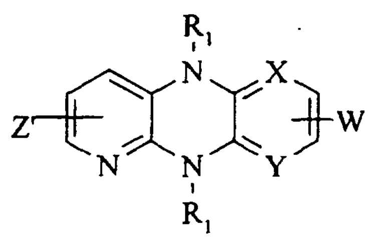

**(I/o)**

dans laquelle X, Y, $R_1$, Z et W ont la même signification que dans la formule (I), et Z' est tel que défini précédemment, étant entendu que les groupements $R_{50}$ et $R_{70}$ définis précédemment ont une longueur de chaîne telle qu'elle permette d'obtenir des composés (I/n) et (I/o) dans lesquels W comporte de 5 à 24 chaînons,
les composés (I/a) à (I/o) formant l'ensemble des composés de l'invention, que l'on purifie, le cas échéant, selon une technique classique de purification, qui peuvent, si on le désire, être séparés en leurs différents isomères selon une technique classique de séparation, et que l'on transforme, le cas échéant en leurs sels d'addition à un acide ou à une base pharmaceutiquement acceptable.

Selon une variante d'obtention des produits de l'invention, les composés de formule (I/a) peuvent, si on le désire, être soumis à une réaction de métallation dans laquelle l'anion intermédiaire est piégé par un dérivé de l'étain, du bore, ou du silicium, permettant d'obtenir les composés de formules ($\alpha$) et ($\beta$), et/ou ($\chi$) :

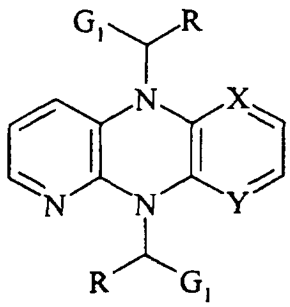

(et/ou)

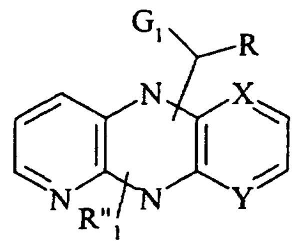

(α) (β)

(et/ou)

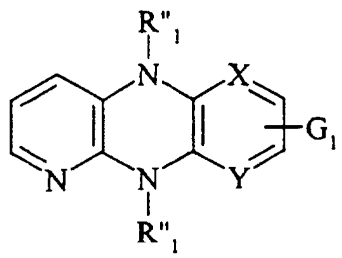

(χ)

dans lesquelles :

- X et Y sont tels que définis dans la formule (I),
- R"$_1$ représente un groupement alkyle ($C_1$-$C_6$) linéaire,
- R représente un atome d'hydrogène ou un groupement alkyle ($C_1$-$C_5$) linéaire,
- et $G_1$ représente un atome d'étain, de bore, ou de silicium, chacun de ces atomes étant substitué par un ou plusieurs groupements alkyle ($C_1$-$C_6$) linéaire ou ramifié.

Ces composés de formule (α), (β) et (χ) sont utiles en tant qu'intermédiaires de synthèse. Ils peuvent, en effet, subir éventuellement une réaction de couplage catalysée par le palladium, celle-ci permettant d'introduire une chaîne latérale alkylée éventuellement substituée. Selon un choix judicieux du réactif de couplage, il est alors possible d'obtenir, à partir de ces intermédiaires et plus spécifiquement à partir des intermédiaires de formule (χ), les composés de formule (I) dans laquelle l'un des groupements $R_2$ ou $R_3$ représentent un groupement Z, avec Z représentant un groupement alkyle ($C_1$-$C_6$) linéaire ou ramifié éventuellement substitué par un ou plusieurs groupements tels que définis dans la formule (I).

Les composés de formules (II), (III), (V), (VII), (VIII) et (IX) sont soit des composés commerciaux, soit obtenus selon les méthodes classiques de synthèse organique.

Les composés de formule (I) présentent des propriétés antitumorales particulièrement intéressantes. Ils ont une excellente cytotoxicité *in vitro* sur des lignées cellulaires, et une action sur le cycle cellulaire. Les propriétés caractéristiques de ces composés permettent leur utilisation en thérapeutique en tant qu'agents antitumoraux.

La présente invention a également pour objet les compositions pharmaceutiques renfermant comme principe actif au moins un composé de formule (I), un de ses isomères optiques ou un de ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les sachets, les gélules, les tablettes, les suppositoires, les crèmes, les pommades, les gels dermiques, les préparations injectables ou buvables, les aérosols, les gouttes oculaires ou nasales, etc...

La posologie utile varie selon l'âge et le poids du patient, la voie d'administration, la nature et la sévérité de l'affection et la prise de traitements éventuels associés et s'échelonne de 1 mg à 250 mg en une ou plusieurs prises par jour.

Les exemples suivants illustrent l'invention mais ne la limitent en aucune façon. Les produits de départ utilisés sont des produits connus ou préparés selon des modes opératoires connus.

Les différentes préparations conduisent à des intermédiaires de synthèse, utiles pour la préparation des composés de l'invention.

Les structures des composés décrits dans les exemples et les préparations ont été déterminées selon les techniques spectrophotométriques usuelles (infrarouge, résonance magnétique nucléaire, spectromètre de masse...).

**Préparation A : 2-amino-3-bromopyridine**

***Stade A* : *N1-(2-pyridyl)-2,2-diméthylpropanamide***

A une solution contenant un équivalent de 2-aminopyridine et 1,25 équivalents de triéthylamine dans 400 ml de dichlorométhane sont additionnés, goutte à goutte à 0°C, 1,1 équivalents de chlorure d'acide pivalique en solution dans 100 ml de dichlorométhane. Après addition complète, la réaction est ramenée à température ambiante pendant trois heures, puis le milieu réactionnel est lavé par une solution saturée en $NaHCO_3$ Après séchage de la phase organique, filtration et concentration sous pression réduite, le résidu obtenu est cristallisé dans l'hexane, permettant d'isoler après filtration le produit attendu.

***Stade B* : *N1-(3-bromo-2-pyridyl)-2,2-diméthylpropanamide***

A une solution de 2,5 équivalents de n-butyllithium 1,6 M en solution dans l'hexane, placée à - 30°C, est additionné goutte à goutte un équivalent du produit obtenu dans le stade A. Après addition complète, la réaction est ramenée à 0°C. Après quatre heures de réaction, la solution est refroidie à - 78°C et 3 équivalents de dibromo-1,2-éthane sont rapidement additionnés. Le mélange réactionnel est ensuite ramené lentement à température ambiante, puis il est hydrolysé et extrait. Les phases organiques sont ensuite séchées sur $MgSO_4$, filtrées et concentrées sous pression résuite. Une chromatographie sur gel de silice (acétate d'éthyle / hexane : 30 / 70) permet d'isoler le produit attendu.

***Stade C* : *2-amino-3-bromopyridine***

Le composé obtenu dans le stade B est mis au reflux pendant 3 heures dans une solution 50 % d'HCl 12N. Le mélange réactionnel est ensuite refroidi, puis lavé à l'éther. La phase aqueuse est ensuite basifiée par addition de potasse puis extraite au dichlorométhane. Les phases organiques sont ensuite séchées sur sulfate de magnésium, filtrées puis concentrées sous pression réduite, permettant d'obtenir le produit attendu.

**Préparation B : N-(3-bromo-2-pyridyl)-N-méthylamine**

A une solution de 1,1 équivalents d'hydrure de sodium dans 60 ml de tétrahydrofurane est additionné à température ambiante 1 équivalent du composé de la préparation A dilué dans 60 ml de tétrahydrofurane. Le mélange réactionnel est ensuite chauffé à 40°C pendant 30 minutes puis 1 équivalent d'iodure de méthyle sont additionnés à -30°C. La solution est alors ramenée à température ambiante pendant 8 heures. Le mélange réactionnel est ensuite jeté sur de la glace puis extrait à l'éther et au dichlorométhane. Les phases organiques sont ensuite lavées à l'eau, séchées sur $MgSO_4$, filtrées et évaporées sous pression réduite. Une chromatographie sur gel de silice (Acétate d'éthyle / Hexane) permet d'isoler le produit attendu.

**Préparation C : N-(3-bromo-2-pyridyl)-N-éthylamine**

On procède comme dans la préparation B en utilisant comme réactif l'iodure d'éthyle. Celui-ci est additionné à température ambiante puis le milieu réactionnel est placé au reflux du tétrahydrofurane pendant 8 heures.

**Préparation D : N-(3-bromo-2-pyridyl)-N-propylamine**

On procède comme dans la préparation B en utilisant comme réactif le 1-bromopropane.

**Préparation E : N-(3-bromo-2-pyridyl)-N-butylamine**

On procède comme dans la préparation D en utilisant comme réactif le 1-bromobutane.

**Préparation F : N1-(3-bromo-2-pyridyl)-N2,N2-diméthyl-1,2-éthanediamine**

On procède comme dans la préparation B en utilisant comme réactif le chlorhydrate de 2-chloro-N,N-diméthyléthylamine. Celui-ci est additionné à 0°C et après addition complète 1 équivalent d'hydrure de sodium est ajouté, puis le milieu réactionnel est porté au reflux du tétrahydrofurane pendant 48 heures.

**Préparation G : N1-(3-bromo-2-pyridyl)-N2,N2-diméthyl-1,3-propanediamine**

On procède comme dans la préparation F en utilisant comme réactif le chlorhydrate de 3-chloro-N,N-diméthylpropylamine.

**EXEMPLE 1 : 5,10-diméthyl-5,10-dihydrodipyrido-[2,3-*b* : 2,3-e]pyrazine**

A 7 équivalents d'amidure de sodium dans 10 ml de tétrahydrofurane (concentration = 70 mM) sont ajoutés, goutte à goutte à température ambiante, 2 équivalents de tert-butanol. La solution est chauffée 2 heures à 45°C, puis refroidie sous courant d'azote à 0°C. On ajoute alors au mélange réactionnel 1 équivalent du composé obtenu dans la préparation B en solution dans 80 ml de tétrahydrofurane (concentration=30 mM). Après 3 jours de réaction à température ambiante, le mélange réactionnel est jeté sur de la glace puis extrait au dichlorométhane. La phase organique est ensuite séchée sur sulfate de magnésium, filtrée et concentrée sous pression réduite. Une chromatographie sur gel de silice (hexane/acétone) permet d'isoler le produit attendu.
*Point de fusion : 152°C.*

**EXEMPLE 2 : 5,10-diméthyl-5,10-dihydrodipyrido-[2,3-*b* : 3,2-*e*]pyrazine**

Le produit est isolé lors de la purification du composé obtenu dans l'exemple 1.
*Point de fusion : 150°C.*

**EXEMPLE 3 : 5,10-diéthyl-5,10-dihydrodipyrido-[2,3-*b* : 2,3-*e*]pyrazine**

On procède comme dans l'exemple 1 en utilisant comme substrat le composé obtenu dans la préparation C.
*Point de fusion : 145°C.*

**EXEMPLE 4 : 5,10-diéthyl-5,10-dihydrodipyrido-[2,3-*b* : 3,2-*e*]pyrazine**

Le produit est isolé lors de la purification du composé obtenu dans l'exemple 3.
*Point de fusion : 131°C.*

**EXEMPLE 5 : 5,10-dibutyl-5,10-dihydrodipyrido-[2,3-*b* : 2,3-e]pyrazine**

On procède comme dans l'exemple 1 en utilisant comme substrat le composé obtenu dans la préparation E.
*Point de fusion : 94°C.*

**EXEMPLE 6 : 5,10-dibutyl-5,10-dihydrodipyrido-[2,3-*b* : 3,2-e]pyrazine**

Le produit est isolé lors de la purification du composé obtenu dans l'exemple 5.
*Point de fusion : 115°C.*

**EXEMPLE 7 : N1,N1-diméthyl-2-{10-[2-(diméthylamino)éthyl]-5,10-dihydrodipyrido[2,3-*b* : 2,3-*e*]pyrazin-5-yl}-1-éthanamine**

On procède comme dans l'exemple 1 en utilisant comme substrat le composé obtenu dans la préparation F.
*Point de fusion : 144°C.*

**EXEMPLE 8 : N1,N1-diméthyl-3-{10-[3-diméthylamino)propyl]-5,10-dihydrodipyrido-[2,3-*b* : 2,3-*e*]pyrazin-5-yl}-1-propanamine**

On procède comme dans l'exemple 1 en utilisant comme substrat le composé obtenu dans la préparation G.
*Point de fusion : 100°C.*

**EXEMPLE 9: N1,N1-diméthyl-3-{10-[3-(diméthylamino)propyl]-5,10-dihydrodipyrido-[2,3-*b* : 3,2-*e*]pyrazin-5-yl}-1-propanamine**

Le produit est isolé lors de la purification du composé obtenu dans l'exemple 8.
*Point de fusion : 84°C.*

**EXEMPLE 10 : 5,10-dipropyl-5,10-dihydrodipyrido-[2,3-*b* : 2,3-e]pyrazine**

A une solution contenant 4 équivalents de n-butyllithium (1,6 M dans l'hexane), dans 30 ml de tétrahydrofurane (concentration=8 mM), refroidie à - 10°C, est additionné le composé obtenu dans l'exemple 1. Après 4 heures de réaction à 0°C, le milieu réactionnel est refroidi à - 30°C et 4 équivalents d'iodure d'éthyle sont ajoutés rapidement. La réaction est alors ramenée à température ambiante, puis hydrolysée sur un mélange eau-glace. Après extraction à l'éther, les phases organiques sont séchées sur $MgSO_4$, filtrées et concentrées sous pression réduite. Une chromatographie sur gel de silice (Hexane/acétate d'éthyle : 90/10) permet d'isoler le produit attendu.
*Point de fusion : 126°C.*

**EXEMPLE 11 : 5-Méthyl-10-propyl-5,10-dihydrodipyrido-[2,3-*b* : 2,3-*e*]pyrazine**

Le produit est isolé lors de la purification du composé obtenu dans l'exemple 10.

**EXEMPLE 12 : 5,10-di-[(méthylthio)méthyl]-5,10-dihydrodipyrido-[2,3-*b* : 2,3-*e*] pyrazine**

A une solution contenant 4 équivalents de n-butyllithium (1,6 M dans l'hexane) dans 30 ml de tétrahydrofurane (concentration=8 mM) sont additionnés à - 70°C, 4 équivalents de tétraméthyléthylènediamine dilués dans 20 ml de tétrahydrofurane (concentration=8 mM). La température est ramenée à - 20°C pendant 1 heure, puis à - 5°C et le composé obtenu dans l'exemple 1 est additionné au milieu réactionnel. Après 4 heures de réaction à 0°C, le milieu est à nouveau refroidi à - 30°C et 4 équivalents de diméthyldisulfure sont ajoutés. La réaction est ensuite ramenée à température ambiante, puis hydrolysée sur un mélange eau-glace. Après extraction à l'éther, les phases organiques sont séchées sur $MgSO_4$, filtrées et concentrées sous pression réduite. Une chromatographie sur gel de silice (Hexane/acétate d'éthyle : 90/10) permet d'isoler le produit désiré.
*Point de fusion :169°C.*

**EXEMPLE 13 : 5-Méthyl-10-[(méthylthio)méthyl]-5,10-dihydrodipyrido-[2,3-*b* : 2,3-e]pyrazine**

Le produit est isolé lors de la purification du composé obtenu dans l'exemple 12.
*Point de fusion : 108°C.*

**EXEMPLE 14 : 5,10-diméthyl-4-(méthylthio)-5,10-dihydrodipyrido-[2,3-*b* :2,3-*e*] pyrazine**

Le produit est isolé lors de la purification du composé obtenu dans l'exemple 12. *Point de fusion : 88°C.*

**EXEMPLE 15 : 5,10-di-(2-méthoxyéthyl)-5,10-dihydrodipyrido-[2,3-*b* : 2,3-e] pyrazine**

On procède comme dans l'exemple 10 en remplaçant l'iodure d'éthyle par du chloro(méthoxy)-méthane.
*Point de fusion : 108°C.*

**EXEMPLE 16 : 5,10-dimétbyl-4-(méthylthio)-5,10-dihydrodipyrido-[2,3-*b* : 3,2-*e*] pyrazine**

On procède comme dans l'exemple 10 en utilisant comme substrat le produit obtenu dans l'exemple 2 et comme réactif le diméthyldisulfure.
*Point de fusion : 99°C.*

**EXEMPLE 17 : 4-méthoxyméthyl-5,10-diméthyl-5,10-dihydrodipyrido-[2,3-*b* : 3,2-*e*] pyrazine**

On procède comme dans l'exemple 16 en utilisant comme réactif électrophile celui utilisé dans l'exemple 15.
*Point de fusion : 95°C.*

**EXEMPLE 18 : 5,10-dibutyl-4-(méthylthio)-5,10-dihydrodipyrido-[2,3-*b* : 3,2-*e*] pyrazine**

On procède comme dans l'exemple 10 en utilisant comme substrat le produit obtenu dans l'exemple 6 et comme réactif le diméthyldisulfure.

**EXEMPLE 19 : 5,10-diméthyl-2-nitro-5,10-dihydrodipyrido-[2,3-*b* : 2,3-e]pyrazine**

A une solution contenant 1 équivalent du composé obtenu dans l'exemple 1 dans 50 ml de dichlorométhane (concentration=6 mM), on additionne à température ambiante de l'acide oxalique. Après 20 heures de réaction, le milieu réactionnel est filtré et le précipité est lavé au dichlorométhane puis séché sous pression réduite. Le produit obtenu est additionné à 50 ml de nitrométhane (concentration=6 mM), puis du nitrite de potassium est ajouté en excès. Après 48 heures de réaction à température ambiante, le mélange réactionnel est jeté sur une solution de potasse 1M. Après extraction au dichlorométhane, les phases organiques rassemblées sont séchées sur sulfate de sodium, filtrées et concentrées sous pression réduite. Une chromatographie sur gel de silice (Hexane / acétate d'éthyle : 50/50) permet d'isoler le produit attendu.
*Point de fusion : 212°C.*

**EXEMPLE 20 : 5,10-dibutyl-2-nitro-5,10-dihydrodipyrido-[2,3-*b* : 2,3-*e*]pyrazine**

On procède comme dans l'exemple 19, en utilisant comme substrat le composé obtenu dans l'exemple 5.
*Point de fusion : 111°C.*

**EXEMPLE 21 : 5,10-dibutyl-2,7-dinitro-5,10-dihydrodipyrido-[2,3-*b* : 2,3-e]pyrazine**

Le produit est isolé lors de la purification du composé obtenu dans l'exemple 20.
*Point de fusion : 221°C.*

**EXEMPLE 22 : N1,N1-diméthyl-2-{10-[2-(diméthylamino)éthyl]-2-nitro-5,10-dihydrodipyrido [2,3-*b* : 2,3-*e*]pyrazin-5-yl}-1-éthylamine**

On procède comme dans l'exemple 19, en utilisant comme substrat le produit obtenu dans l'exemple 7.
*Point de fusion : 110°C.*

**EXEMPLE 23 : N1,N1-diméthyl-3-{10-[3-diméthylamino)propyl]-2-nitro-5,10-dihydrodipyride-[2,3-*b* : 2,3-*e*]pyrazin-5-yl}-1-propanamine**

On procède comme dans l'exemple 19, en utilisant comme substrat le produit obtenu dans l'exemple 8. *Point de fusion : 95°C.*

**EXEMPLE 24 : N1,N1-diméthyl-3-{10-[3-(diméthylamino)propyl]-2,7-dinitro-5,10-dihydrodipyrido-[2,3-*b* : 2,3-*e*] pyrazin-5-yl}-1-propanamine**

Le produit est isolé lors de la purification du composé obtenu dans l'exemple 23.

**EXEMPLE 25 : 5,10-diméthyl-2-nitro-5,10-dihydrodipyrido-[2,3-*b* : 3,2-*e*]pyrazine**

On procède comme dans l'exemple 19 en utilisant comme substrat le composé obtenu dans l'exemple 2.

**EXEMPLE 26 : 5,10-diméthyl-2,8-dinitro-5,10-dihydrodipyrido-[2,3-*b* : 3,2-*e*] pyrazine**

Le produit est isolé lors de la purification du composé obtenu dans l'exemple 25.
*Point de fusion : > 270°C.*

**EXEMPLE 27 : N1,N1-diméthyl-3-{10-[3-(diméthylamino)propyl] 2-nitro-5,10-dihydrodipyrido-[2,3-*b* : 3,2-*e*]pyrazin-5-yl}-1-propanamine**

On procède comme dans l'exemple 19, en utilisant comme substrat le composé obtenu dans l'exemple 9.
*Point de fusion : 123°C.*

**EXEMPLE 28 : N1,N1-diméthyl-3-{10-[3-diméthylamino)propyl] 2,8-dinitro-5,10-dihydrodipyrido-[2,3-*b* : 3,2-*e*] pyrazin-5-yl}-1-propanamine**

Le produit est isolé lors de la purification du composé obtenu dans l'exemple 27.
*Point de fusion : 160-170°C.*

**EXEMPLE 29 : 5,10-dibutyl-2,8-dinitro-5,10-dihydrodipyrido-[2,3-*b* : 3,2-*e*]pyrazine**

On procède comme dans l'exemple 19 en utilisant comme substrat le composé obtenu dans l'exemple 6.
*Point de fusion : 202°C.*

**EXEMPLE 30 : 2-amino-5,10-diméthyl-5,10-dihydrodipyrido-[2,3-*b* : 2,3-e]pyrazine**

Une solution contenant 1 équivalent du composé obtenu dans l'exemple 19 dans 50 ml de méthanol (concentration=1 mM), 100 mg de palladium sur charbon à 10 % et 4,6 équivalents de formiate d'ammonium est chauffée pendant 3 heures à 40°C. Après retour à température ambiante, le milieu réactionnel est filtré sur célite, puis concentré sous pression réduite permettant d'isoler le produit désiré.
*Point de fusion : 134°C.*

**EXEMPLE 31 : N1,N1-diméthyl-2-{10-[2-(diméthylamino)éthyl]-2-amino-5,10-dihydrodipyrido[2,3-*b* : 2,3-*e*]pyrazin-5-yl}-1-éthylamine**

On procède comme dans l'exemple 30 en utilisant comme substrat le composé obtenu dans l'exemple 22.
*Point de fusion : 139°C.*

**EXEMPLE 32 : N1,N1-diméthyl-3-{10-[3-(diméthylamino)propyl]-2-amino-5,10-dihydrodipyrido [2,3-*b* : 2,3-*e*] pyrazin-5-yl}-1-propanamine**

On procède comme dans l'exemple 30 en utilisant comme substrat le composé obtenu dans l'exemple 23.
*Point de fusion : 98°C.*

**EXEMPLE 33 : 2-amino-5,10-diméthyl-5,10-dihydrodipyrido-[2,3-*b* :3,2-e]pyrazine**

On procède comme dans l'exemple 30 en utilisant comme substrat le composé obtenu dans l'exemple 25.
*Point de fusion : 124°C.*

**EXEMPLE 34 : N1,N1-diméthyl-3-{10-[3-(diméthylamino)propyl]-2-amino-5,10-dihydrodipyrido[2,3-*b* : 3,2-*e*]pyrazin-5-yl} 1-propanamine.**

On procède comme dans l'exemple 30 en utilisant comme substrat le composé obtenu dans l'exemple 27.

**EXEMPLE 35 : N1-(5,10-diméthyl-5,10-dihydrodipyrido [2,3-*b* : 3,2-*e*]pyrazin-2-yl)-1-benzènesulfonamide.**

A une solution de 3 équivalents de pyridine dans 25 ml de dichlorométhane (concentration=3 mM) placée à - 20°C sont additionnés goutte à goutte 1,2 équivalents de chlorure de benzène sulfonyle dilué dans 10 ml de dichlorométhane (concentration=1 mM) Après 1 heure de réaction, 1 équivalent du composé obtenu dans l'exemple 33 est additionné, puis le milieu réactionnel est ramené à température ambiante. Après 12 heures, la réaction est hydrolysée, puis extraite au dichlorométhane. Les phases organiques rassemblées sont séchées sur sulfate de sodium, filtrées et concentrées sous pression réduite. Une chromatographie sur gel de silice (Hexane/acétate d'éthyle : 60/40) permet d'isoler le produit attendu.
*Point de fusion : 208°C.*

**EXEMPLE 36 : 5,10-diméthyl-5,10-dihydrodipyrido [2,3-*b* : 2,3-*e*]pyrazine-2-carbaldéhyde**

A 23 équivalents de diméthylformamide refroidis à 0°C sont additionnés goutte à goutte 10 équivalents de $POCl_3$, puis la réaction est ramenée à température ambiante. Après 20 minutes, cette solution est additionnée goutte à goutte à une solution composée de un équivalent du composé obtenu dans l'exemple 1 dilué dans du dichloro-1,2-éthane. L'addition s'effectue au reflux du dichloro-1,2-éthane et celui-ci est maintenu pendant 20 heures. Le milieu

réactionnel est ensuite refroidi à 0°C, puis on additionne une solution de potasse 3 M (120 ml) et le milieu est chauffé au reflux. Après 3 heures, la réaction est refroidie et extraite au dichlorométhane. Les phases organiques rassemblées sont ensuite lavées à l'eau, séchées sur sulfate de magnésium, filtrées et concentrées sous pression réduite. Une chromatographie sur gel de silice (Hexane/acétate d'éthyle 70/30) permet d'isoler le produit attendu. *Point de fusion : 176°C.*

**EXEMPLE 37 : 5,10-diméthyl-5,10-dihydrodipyrido [2,3-*b* : 2,3-*e*]pyrazine-3-carbaldéhyde**

Le produit est isolé lors de la purification du composé obtenu dans l'exemple 36.

**EXEMPLE 38 : 5,10-diméthyl-5,10-dihydrodipyrido [2,3-*b* : 3,2-*e*] pyrazine-2-carbaldéhyde**

On procède comme dans l'exemple 36 en utilisant comme substrat le composé obtenu dans l'exemple 2. *Point de fusion : 157°C.*

**EXEMPLE 39 : N1-[(5,10-diméthyl-5,10-dihydrodipyrido[2,3-*b* : 2,3-*e*]pyrazin-2-yl)méthyl]-N2,N2-diméthyl-1,2-éthanediamine**

A une solution de 1 équivalent du composé obtenu dans l'exemple 36 dans 25 ml de dichlorométhane (concentration=2mM) sont additionnés rapidement 3,6 équivalents de N,N-diméthyléthylènediamine dilués dans 25 ml de dichlorométhane (concentration=6mM). Après une heure à température ambiante, le solvant est évaporé sous pression réduite et remplacé par du méthanol. 10 équivalents de $NaBH_4$ sont alors additionnés lentement dans le milieu réactionnel. Après 15 minutes de réaction, le milieu est jeté sur de la glace, puis extrait au dichlorométhane. Les phases organiques sont séchées sur sulfate de magnésium, filtrées et évaporées sous pression réduite. Une chromatogaphie sur gel de silice (triéthylamine/méthanol : 50/50) permet d'isoler le produit attendu.

**EXEMPLE 40 : N1-[(5,10-diméthyl-5,10-dihydrodipyrido [2,3-*b* : 3,2-*e*] pyrazin-2-yl)méthyl]-N2,N2-diméthyl-1,2-éthanediamine**

On procède comme dans l'exemple 39 en utilisant comme substrat le composé obtenu dans l'exemple 38. *Point de fusion : huile.*

**EXEMPLE 41 : 2-bromo-5,10-dibutyl-5,10-dihydrodipyrido-[2,3-*b* : 3,2-*e*] pyrazine**

A une solution d'un équivalent du composé obtenu dans l'exemple 6 dilué dans 50 ml de tétrahydrofurane (concentration=2mM) sont additionnés, à température ambiante, 1,1 équivalents de tribromure de phényltriméthylammonium en solution dans 50 ml de tétrahydrofurane (concentration=2mM). Après 24 heures de réaction, le milieu réactionnel est hydrolysé puis extrait à l'éther. Les phases organiques rassemblées sont séchées sur $MgSO_4$, filtrées et concentrées sous pression réduite Une chromatographie sur gel de silice (Hexane/acétate d'éthyle : 98/2) permet d'isoler le produit attendu.

**EXEMPLE 42 : 2,8-dibromo-5,10-dibutyl-5,10-dihydrodiyprido-[2,3-*b* : 3,2-e] pyrazine**

Le produit est isolé lors de la purification du composé obtenu dans l'exemple 41. *Point de fusion : huile.*

**EXEMPLE 43 : N1,N2-di[(5,10-diméthyl-5,10-dihydrodipyrido-[2,3-*b* : 3,2-*e*]pyrazin-2-yl)méthyl]-1,2-éthanediamine**

A une solution de deux équivalents du composé obtenu dans l'exemple 38 dilués dans 25 ml de dichlorométhane (concentration=2 mM) est additionné un équivalent de 1,2-diaminoéthane dilué dans 25 ml de dichlorométhane (concentration=2 mM). Après 30 minutes de réaction à température ambiante, la solution est concentrée sous pression réduite puis le résidu est dilué dans 25 ml de méthanol (concentration=2 mM). 10 équivalents de $NaBH_4$ sont alors additionnés lentement, puis après 30 minutes, la réaction est hydrolysée à 0°C. Après extraction au dichlorométhane, les phases organiques rassemblées sont séchées sur sulfate de magnésium, filtrées et concentrées sous pression réduite. Le résidu est ensuite élué au méthanol sur gel de silice. Les fractions sont évaporées sous pression réduite puis reprises au dichlorométhane et de nouveau évaporées sous pression réduite, permettant ainsi d'isoler le produit attendu.

*Point de fusion : 171°C.*

**EXEMPLE 44 : N1,N3-di [(5,10-diméthyl-5,10-dihydrodipyrido [2,3-*b* : 3,2-*e*] pyrazin-3-yl)méthyl]-1,3-propanediamine**

On procède comme dans l'exemple 43, en utilisant comme réactif le 1,3-diaminopropane. *Point de fusion : 61°C.*

**EXEMPLE 45 : N1,N2-di[(5,10-diméthyl-5,10-dihydrodipyrido-(2,3-*b* : 2,3-*e*]pyrazin-2-yl)méthyl]-1,2-éthanediamine**

On procède comme dans l'exemple 43, en utilisant comme substrat le composé obtenu dans l'exemple 37 *Point de fusion : 80-90°C.*

**EXEMPLE 46 : N1-[(E)-1-(5,10-diméthyl-5,10-dihydrodipyrido-[2,3-*b* : 2,3-*e*]pyrazin-2-yl)méthylidène]-N2-[(Z)-1-(5,10-diméthyl-5,10-dihydrodipyrido [2,3-*b* : 2,3-*e*]pyrazin-2-yl)méthylidène]-1,2-éthanediamine**

Le produit est obtenu lors de la synthèse du composé de l'exemple 45, lorsque la réaction est arrêtée avant le traitement par le borohydrure de sodium.

**EXEMPLE 47 : N1-[(E)-1-(5,10-diméthyl-5,10-dihydrodipyrido-[2,3-*b* : 3,2-*e*] pyrazin-2-yl)méthylidène]-N3-[(Z)-1-(5,10-diméthyl-5,10-dihydrodipyrido[2,3-*b* : 3,2-*e*]pyrazin-2-yl)méthylidène]-1,3-propanediamine**

Le produit est obtenu lors de la synthèse du composé de l'exemple 44, lorsque la réaction est arrêtée avant le traitement par le borohydrure de sodium.

**EXEMPLE 48 : N1-[(E)-1-(5,10-diméthyl-5,10-dihydrodipyrido-[2,3-*b* : 3,2-*e*]pyrazin-2-yl)méthylidène]-N2-[(Z)-1-(5,10-diméthyl-5,10-dihydrodipyrido[2,3-*b* : 3,2-*e*]pyrazin-2-yl)méthylidène]-1,2-éthanediamine**

Le produit est obtenu lors de la synthèse du composé de l'exemple 43, lorsque la réaction est arrêtée avant le traitement par le borohydrure de sodium.

**EXEMPLE 49 : N1,N4-di[(5,10-diméthyl)-5,10-dihydrodipyrido[2,3-*b* : 3,2-*e*]pyrazin-2-yl)méthyl]-1,4-butanediamine**

On procède comme dans l'exemple 43, en utilisant comme réactif le 1,4-diaminobutane.

**EXEMPLE 50 : N1-[(E)-1-(5,10-diméthyl-5,10-dihydrodipyrido[2,3-*b* : 3,2-*e*]pyrazin-2-yl)méthylidène]-N4-[(Z)-1-(5,10-diméthyl-5,10-dihydrodipyrido [2,3-*b* : 3,2-*e*]pyrazin-2-yl)méthylidène]-1,4-butanediamine**

Le produit est obtenu lors de la synthèse du composé de l'exemple 49, lorsque la réaction est arrêtée avant le traitement par le borohydrure de sodium.

**EXEMPLE 51 : 5,10-dibutyl-5,10-dihydrodipyrido[2,3-*b* : 3,2-*e*]pyrazine-2-carbaldéhyde**

On procède comme dans l'exemple 36 en utilisant comme substrat le composé obtenu dans l'exemple 6. *Point de fusion* : 108°C.

**EXEMPLE 52 : 5,10-dibutyl-5,10-dihydrodipyrido[2,3-*b* : 2,3-*e*]pyrazine-2-carbaldéhyde**

On procède comme dans l'exemple 36 en utilisant comme substrat le composé de l'exemple 5. *Point de fusion : 95°C.*

**EXEMPLE 53 : 5,10-dibutyl-2-nitro-5,10-dihydrodipyrido-[2,3-*b* : 3,2-*e*]pyrazine**

Le produit est isolé lors de la purification du composé obtenu dans l'exemple 29. *Point de fusion : 128°C.*

**EXEMPLE 54 : 2-[10-(2-hydroxy-2-phényléthyl)-5,10-dihydrodipyrido-[2,3-*b* : 2,3-*e*] pyrazin-5-yl]-1-phényl-1-éthanol**

On procède comme dans l'exemple 12 en utilisant comme réactif le benzaldéhyde à la place du diméthyldisulfide.
*Point de fusion : 189°C.*

**EXEMPLE 55 : (5,10-diméthyl-5,10-dihydrodipyrido[2,3-*b* : 3,2-e]pyrazin-4-yl] (phényl)méthanol**

On procède comme dans l'exemple 54 en utilisant comme substrat le composé de l'exemple 2.
*Point de fusion : 126°C.*

**EXEMPLE 56 : 5,10-diméthyl-5,10-dihydrodipyrido[2,3-*b* : 3,2-*e*]pyrazin-4-carbaldéhyde**

A une solution de 0,94 mmol de n-butyllithium (1,6 M dans l'hexane) dans 2 ml de tétrahydrofurane à -10°C sont additionnés 0,47 mmol du composé de l'exemple 2. Après une heure de réaction à -10°C, 2 à 5 équivalents de diméthylformamide sont ajoutés. La réaction est ensuite ramenée à température ambiante, hydrolysée puis extraite au dichlorométhane. Après séchage, filtration et concentration sous pression réduite des phases organiques, une chromatographie sur gel de silice (acétate d'éthyle/hexane : 10/90) du résidu permet d'isoler le produit attendu.
*Point de fusion : 184-185°C.*

**EXEMPLE 57 : N1,N3-di[(5,10-diméthyl-5,10-dihydrodipyrido-[2,3-*b* : 3,2-*e*]pyrazin-4-yl)méthyl]-1,3-propanediamine**

On procède comme dans l'exemple 43 en utilisant comme substrat le composé de l'exemple 56 et comme réactif le 1,3-diaminopropane.
*Spectrométrie de Masse : (spray ionique) : [M+1] : m/z = 523,5*

**EXEMPLE 58 : N1,N7-di[(5,10-diméthyl-5,10-dihydrodipyrido-[2,3-*b* : 3,2-e]pyrazin-2-yl)méthyl]-1,7-heptanediamine**

On procède comme dans l'exemple 43 en utilisant comme réactif le 1,7-diaminoheptane. *Spectométrie de Masse : (spray ionique) : [M+1] : m/z = 551,5*

**EXEMPLE 59 : N1-[(5,10-diméthyl-5,10-dihydrodipyrido[2,3-*b* : 3,2-*e*]pyrazin-2-yl) méthyl]-N3-(3-{[5,10-diméthyl-5,10-dihydrodipyrido[2,3-*b* : 3,2-*e*] pyrazin-2-yl)méthyl]amino}propyl)-N3-méthyl-1,3-propanediamine**

On procède comme dans l'exemple 43 en utilisant comme réactif le N,N-bis(3-aminopropyl)-N-méthylamine.
*Spectrométrie de Masse : (spray ionique) : [M+1] : m/z = 594,5*

**EXEMPLE 60 : N1,N3-di[(5,10-diméthyl-5,10-dihydrodipyrido-[2,3-*b* : 3,2-e]pyrazin-2-yl]-N1,N3-diméthyl-1,3-propanediamine**

On procède comme dans l'exemple 43 en utilisant comme réactif le N1,N3-diméthyl-1,3-propanediamine.
*Spectrométrie de Masse : (spray ionique) : [M+1] : m/z = 551,5*

**EXEMPLE 61 : N-[(5,10-diméthyl-5,10-dihydrodipyrido-[2,3-*b* : 3,2-*e*]pyrazin-2-yl) méthyl]-2-(2-{[(5,10-diméthyl-5,10-dihydrodipyrido]2,3-b : 3,2-e] pyrazin-2-yl)méthyl]amino}éthoxy)-1-éthanamine**

On procède comme dans l'exemple 43 en utilisant comme réactif le 2-(2-aminoéthoxy)-1-éthanamine.

**EXEMPLE 62 : 5,10-Diméthyl-4-(triméthylsilyl)-5,10-dihydrodipyrido[2,3-b : 3,2-e] pyrazine**

On procède comme dans l'exemple 56 en utilisant comme réactif le chlorure de triméthylsilyle à la place du diméthylformamide.
*Spectrométrie de Masse : (spray ionique) : [M+1] : m/z = 285*

**EXEMPLE 63 : 5,10-Diméthyl-4-(triméthylstannyl)-5,10-dihydrodipyrido [2,3-b : 3,2-e]pyrazine**

On procède comme dans l'exemple 56 en utilisant comme réactif le chlorure de triméthylétain à la place du diméthylformamide.
*Spectrométrie de Masse : (spray ionique) :*

*[M+1, $Sn^{116}$] : m/z = 373*
*[M+1, $Sn^{118}$] : m/z = 375*
*[M+1, $Sn^{120}$] : m/z = 377*

**EXEMPLE 64 : (5,10-Diméthyl-5,10-dihydrodipyrido[2,3-b : 3,2-e] pyrazin-4-yl) (diphényl)méthanol**

On procède comme dans l'exemple 56 en utilisant comme réactif le benzophénone à la place du diméthylformamide. *Spectrométrie de Masse : (spray ionique) : [M+1] : m/z) = 395,5*

**EXEMPLE 65 : 5,10-Diméthyl-5,10-dihydrodipyrido-4-iodo[2,3-b : 3,2-e] pyrazine**

On procède comme dans l'exemple 56 en utilisant comme réactif l'iode à la place du diméthylformamide.
*Spectrométrie de Masse : (spray ionique) : [M+ 1] : m/z = 339*

**EXEMPLE 66 : 5,10-Diméthyl-5,10-dihydrodipyrido[2,3-*b*:3,2-*e*]pyrazine-4-carboxylate de sodium**

A une solution de 0,94 mmol de n-butyllithium (1,6M dans l'hexane) dans 2 ml de tétrahydrofurane, à -10°C, sont additionnés 0,47 mmol du composé de l'exemple 2. Après une heure de réaction à -10°C, le milieu réactionnel est placé sous courant de dioxyde de carbone pendant 20 minutes. Après retour à température ambiante, la réaction est hydrolysée, extraite au dichlorométhane. Une évaporation sous pression réduite de la phase aqueuse permet d'isoler le produit attendu.

**EXEMPLE 67 : 5,10-Diméthyl-5,10-dihydrodipyrido[2,3-b:3,2-e]pyrazine-4-carboxylate d'éthyle**

A une solution de 0,94 mmol de n-butyllithium (1,6M dans l'hexane) dans 2 ml de tétrahydrofurane, à -10°C, sont additionnés 0,47 mmol du composé de l'exemple 2. Après une heure de réaction à -10°C, 2 à 5 équivalents de chloroformiate d'éthyle sont ajoutés. Après retour à température ambiante, le milieu réactionnel est hydrolysé puis extrait au dichlorométhane. Après séchage, filtration et concentration sous pression réduite, une chromatographie sur gel de silice (acétate d'éthyle/hexane : 95/5) permet d'isoler le produit attendu.
*Spectrométrie de Masse : (spray ionique) : [M+1] : m/z = 285,5*

**EXEMPLE 68 : 5,10-Diméthyl-N-propyl-5,10-dihydrodipyrido-[2,3-*b*:3,2-*e*]pyrazine-4-carboxamide**

A une solution de 0,39 mmol du composé de l'exemple 66, 0,37 mmol de propylamine, 0,43 mmol de N-hydroxybenzotriazole dissous dans 4 ml de diméthylformamide, et 0,49 mmol de triéthylamine, sont additionnés à 0°C 0,43 mmol d'hydrochlorure de 1-éthyl-3-(3'-diméthylaminopropyl)-carbodiimide. Après 12 heures de réaction à température ambiante, le milieu réactionnel et hydrolysé, puis extrait au dichlorométhane. Les phases organiques sont lavées par une solution aqueuse à 5 % de $NaHCO_3$, séchées, filtrées puis concentrées sous pression réduite. Une chromatographie sur gel de silice (acétate d'éthyle/hexane : 95/5) permet d'isoler le produit attendu.

**EXEMPLE 69 : N-{2-[2-{[(5,10-diméthyl-5,10-dihydrodipyrido[2,3-*b*:3,2-*e*]pyrazin-4-yl)carbonyl]amino}éthyl) (méthyl)amino]éthyl}-5,10-diméthyl-5,10-dihydrodipyrido-[2,3-*b*:3,2-*e*]pyrazine-4-carboxamide**

On procède comme dans l'exemple 68 en utilisant deux équivalents du composé de l'exemple 2 pour un équivalent de composé aminé, et en remplaçant la propylamine par la N,N-bis(2-aminoéthyl)-N-méthylamine.

***Etudes pharmacologiques des composés de l'invention***

**EXEMPLE 70 : Cytotoxicité des composés**

Trois lignées cellulaires ont été utilisées :

- une leucémie murine, L1210,
- un mélanome murin, B16,
- un carcinome pulmonaire humain, non à petites cellules, A549.

Les cellules sont cultivées dans du milieu RPMI 1640 complet contenant 10 % de sérum de veau foetal, 2mM de glutamine, 50 unités/ml de pénicilline, 50 µg/ml de streptomycine et 10 mM d'Hépès.
Les cellules sont réparties dans des microplaques et exposées aux composés cytotoxiques. Elles sont ensuite incubées pendant 2 jours (L1210) et 4 jours (A549, B16). Le nombre de cellules viables est ensuite quantifié par un essai colorimétrique, le Microculture Tetrazolium Assay (Cancer Res., 1987, 47, 936-942). Les résultats sont exprimés en $IC_{50}$, concentration en cytotoxique qui inhibe à 50 % la prolifération des cellules traitées. Les résultats obtenus montrent une bonne cytotoxicité générale sur les trois lignées cellulaires des composés de l'invention, avec des $IC_{50}$ inférieures à 10 µM.

**EXEMPLE 71 : Activité in vivo**

- * *Activité antitumorale des composés sur la leucémie P388*
  La lignée P388 (leucémie murine) a été fournie par le National Cancer Institute (Frederick, USA). Les cellules tumorales ($10^6$ celules) ont été inoculées au jour 0 dans la cavité péritonéale de souris BDF1 femelles (Iffa-Credo, France) pesant de 18 à 20 g (groupes de 6 animaux). Les produits ont été administrés au jour 1 ou aux jours 1, 5, 9 par voie intrapéritonéale ou intraveineuse.
  L'activité antitumorale est exprimée en % de T/C :

$$\% \text{ T/C} = \frac{\text{Temps de survie médian des animaux traités}}{\text{Temps de survie médian des animaux contrôles}} \times 100$$

  A titre indicatif, le composé de l'exemple 44 est actif sur la leucémie P388 : selon le schéma d'administration, il induit un T/C de 150 à 190 % entre 25 et 50 mg/kg.

- * *Activité* antitumorale *des composés sur le mélanome B16*
  La lignée B16 (mélanome murin) a été fournie par le National Cancer Institute (Frederick, USA). Cette tumeur est maintenue par greffes successives de fragments tumoraux en sous-cutané. Au jour 0, les tumeurs sont broyées et homogénéisées dans du NaCl à 0,9 % (1 g de tumeur dans 10 ml), et 0,5 ml de l'homogénat est injecté dans la cavité péritonéale de chaque souris BDF1. Les produits ont été administrés 1 fois par jour pendant 9 jours, (J1-9) par voie intra-péritonéale, à des groupes de 7 animaux.
  L'activité antitumorale est exprimée en % de T/C.
  Le composé de l'exemple 44 est actif sur le mélanome B16 : il induit un T/C de 140 à 150 % entre 1,56 et 12,5 mg/kg.

**EXEMPLE 72 : Composition pharmaceutique : comprimés**

Formule de préparation pour 1000 comprimés dosés à 50 mg.

| | |
|---|---|
| Composé de l'exemple 44 | 5 g |
| Lactose | 40 g |
| Stéarate de Magnésium | 10 g |
| Amidon de blé | 15 g |
| Amidon de maïs | 15 g |
| Silice | 3 g |
| Hydroxypropylcellulose | 5 g. |

**Revendications**

**1.** Composés de formule (I) :

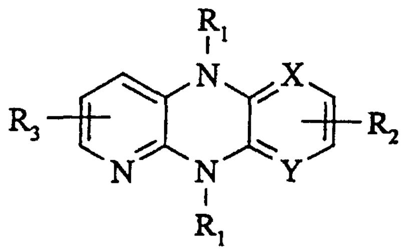

(I)

dans laquelle :

**X** représente un atome d'azote, de carbone, ou un groupement CH,

**Y** représente un atome d'azote quand X représente un atome de carbone ou un groupement CH, ou Y représente un atome de carbone, ou un groupement CH, quand X représente un atome d'azote,

étant entendu que lorsque X et Y représente un atome de carbone alors celui-ci porte le groupement $R_2$,

**$R_1$** représente un groupement alkyle ($C_1$-$C_6$) linéaire ou ramifié (éventuellement substitué par un ou plusieurs groupements, identiques ou différents, choisis parmi halogène, alkylthio ($C_1$-$C_6$) linéaire ou ramifié, alkoxy ($C_1$-$C_6$) linéaire ou ramifié, monoalkyle ou dialkylamino ($C_1$-$C_6$) linéaire ou ramifié, aryle, hydroxy, formyle, alkyloxycarbonyle ($C_1$-$C_6$) linéaire ou ramifié, et carboxy), étant entendu que les deux groupements $R_1$ ont soit des définitions de groupements identiques, soit des définitions de groupements différentes,

**$R_2$ et $R_3$,** identiques ou différents, indépendamment l'un de l'autre, représentent un groupement Z,
ou
l'un desdits groupements $R_2$ ou $R_3$ représente un groupement W et l'autre desdits groupements $R_2$ ou $R_3$ représente un groupement Z,
groupements Z et W dans lesquels :

- Z représente :
  * un atome d'hydrogène,
  * d'halogène,
  * un groupement alkyle ($C_1$-$C_6$) linéaire ou ramifié, ledit groupement alkyle pouvant être substitué par un ou plusieurs groupements, identiques ou différents, choisis parmi hydroxy, alkoxy ($C_1$-$C_6$) linéaire ou ramifié, aryle, amino (éventuellement substitué par un ou deux groupements, identiques ou différents, choisis parmi alkyle ($C_1$-$C_6$) linéaire ou ramifié, aryle, arylalkyle ($C_1$-$C_6$) linéaire ou ramifié, aminoalkyle ($C_1$-$C_6$) linéaire ou ramifié, monoalkylaminoalkyle ($C_1$-$C_6$) linéaire ou ramifié, dialkylaminoalkyle ($C_1$-$C_6$) linéaire ou ramifié, hydroxyalkyle ($C_1$-$C_6$) linéaire ou ramifié, et alkoxyalkyle ($C_1$-$C_6$) linéaire ou ramifié), et formyle,
  * amino (éventuellement substitué par un ou deux groupements, identiques ou différents, choisis parmi alkyle ($C_1$-$C_6$) linéaire ou ramifié, aryle, arylalkyle ($C_1$-$C_6$) linéaire ou ramifié, alkylsulfonyle ($C_1$-$C_6$) linéaire ou ramifié, arylsulfonyle, aminoalklyle ($C_1$-$C_6$) linéaire ou ramifié, monoalkylaminoalkyle ($C_1$-$C_6$) linéaire ou ramifié, dialkylaminoalkyle ($C_1$-$C_6$) linéaire ou ramifié, hydroxyalkyle ($C_1$-$C_6$) linéaire ou ramifié, et alkoxyalkyle ($C_1$-$C_6$) linéaire ou ramifié),
  * nitro,
  * alkylthio ($C_1$-$C_6$) linéaire ou ramifié,
  * formyle,
  * hydroxycarbonyle,
  * alkoxycarbonyle ($C_1$-$C_6$) linéaire ou ramifié,
  * aminocarbonyle, monoalkylaminocarbonyle ($C_1$-$C_6$) linéaire ou ramifié, ou un groupement dialkylaminocarbonyle ($C_1$-$C_6$) linéaire ou ramifié,

- W représente une chaîne alkylène ($C_5$-$C_{24}$) linéaire ou ramifiée, dans laquelle un ou plusieurs atomes de carbone sont éventuellement remplacés par un ou plusieurs groupements, identiques

ou différents, indépendamment l'un de l'autre, choisis parmi atomes d'oxygène, groupements imine, $-N(R_4)-CO-$, $-CO-N(R_4)-$, et $N(R_4)$ dans lesquels $R_4$ représente un atome d'hydrogène, un groupement alkyle $(C_1-C_6)$ linéaire ou ramifié, aryle, ou arylalkyle $(C_1-C_6)$

linéaire ou ramifié, ladite chaîne alkylène étant substituée dans sa position terminale par l'un quelconque des groupements suivants :

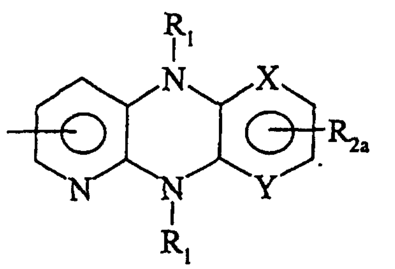

,

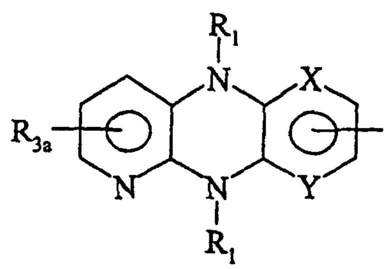

dans lesquels X, Y et $R_1$ sont tels que définis précédemment, et $R_{2a}$ et $R_{3a}$ représentent un groupement Z tel que défini précédemment,

leurs isomères ainsi que leurs sels d'addition à un acide ou à une base pharmaceutiquement acceptable,
étant compris que par groupement aryle, on entend un groupement phényle, naphtyle, tétrahydronapthyle, dihydronaphtyle, indényle ou indanyle, chacun de ces groupements étant éventuellement substitué de façon identique ou différente, par un ou plusieurs atomes d'halogène, groupements hydroxy, cyano, nitro, alkyle $(C_1-C_6)$ linéaire ou ramifié, trihalogénoalkyle $(C_1-C_6)$ linéaire ou ramifié, alkoxy $(C_1-C_6)$ linéaire ou ramifié, acyle $(C_1-C_6)$ linéaire ou ramifié, carboxy, alkoxycarbonyle $(C_1-C_6)$ linéaire ou ramifié, ou amino (substitué éventuellement par un ou deux groupements, identiques ou différents, choisis parmi alkyle $(C_1-C_6)$ linéaire ou ramifié, aryle et arylakyle $(C_1-C_6)$ linéaire ou ramifié).

**2.** Composés de formule (I) selon la revendication 1 **caractérisés en ce que** $R_2$ et $R_3$, identiques ou différents, indépendamment l'un de l'autre, représentent un groupement Z, leurs isomères ainsi que leurs sels d'addition à un acide ou à une base pharmaceutiquement acceptable.

**3.** Composés de formule (I) selon la revendication 1 **caractérisés en ce que** l'un des groupements $R_2$ ou $R_3$ représente un groupement Z et l'autre des groupements $R_2$ ou $R_3$ représente un groupement W, leurs isomères ainsi que leurs sels d'addition à un acide ou à une base pharmaceutiquement acceptable.

**4.** Composés de formule (I) selon l'une quelconque des revendications 1 ou 3 **caractérisés en ce que** l'un des groupements $R_2$ ou $R_3$ représente un groupement Z et l'autre des groupements $R_2$ ou $R_3$ représente un groupement $W_1$, ledit groupement $W_1$ représentant une chaîne alkylène $(C_6-C_{12})$ linéaire dans laquelle deux ou trois atomes de carbone sont remplacés par deux ou trois groupements, identiques ou différents, $N(R_4)$ dans lesquels $R_4$ représente un atome d'hydrogène ou un groupement alkyle $(C_1-C_6)$ linéaire ou ramifié, ladite chaîne alkylène étant substituée dans sa position terminale par l'un quelconque des deux groupements suivants :

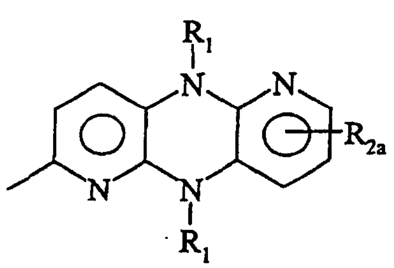

,

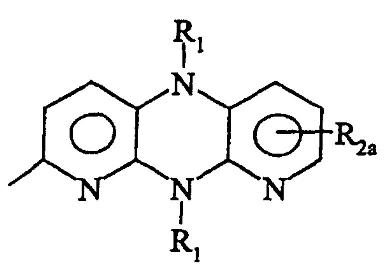

dans lesquels $R_1$ est tel que défini dans la formule générale (I) et $R_{2a}$ représente un groupement Z, leurs isomères ainsi que leurs sels d'addition à un acide ou à une base pharmaceutiquement acceptable.

**5.** Composés de formule (I) selon l'une quelconque des revendications 1 ou 3 **caractérisés en ce que** l'un des groupements $R_2$ ou $R_3$ représente un groupement Z et l'autre des groupements $R_2$ ou $R_3$ représente un groupement $W_2$ de formule :

$$-CO-N(R_4)-W'_2-N(R_4)-CO-G$$

dans laquelle :

- chacun des groupements $R_4$, identiques ou différents, représente un atome d'hydrogène ou un groupement alkyle ($C_1$-$C_6$) linéaire ou ramifié,
- $W'_2$ représente une chaîne alkylène ($C_3$-$C_{12}$) linéaire, dans laquelle un, deux ou trois atomes de carbone sont éventuellement remplacés par un, deux ou trois groupements, identiques ou différents, $N(R_4)$ dans lesquels $R_4$ représente un atome d'hydrogène ou un groupement alkyle ($C_1$-$C_6$) linéaire ou ramifié,
- et G représente l'un quelconque des deux groupements suivants :

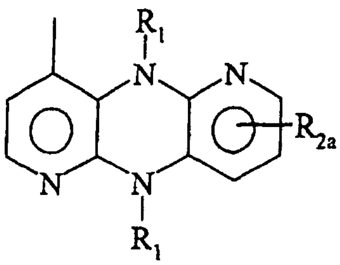
,
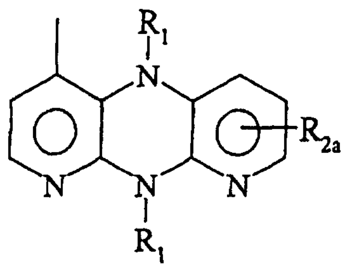

dans lesquels $R_1$ est tel que défini dans la formule générale (I) et $R_{2a}$ représente un groupement Z, leurs isomères ainsi que leurs sels d'addition à un acide ou à une base pharmaceutiquement acceptable.

**6.** Composés de formule (I) selon la revendication 4 **caractérisés en ce que** :

- l'un des groupements $R_2$ ou $R_3$ représente un groupement $W_1$ dans lequel le groupement $R_1$ représente un groupement alkyle ($C_1$-$C_6$) linéaire ou ramifié, et le groupement $R_{2a}$ représente un atome d'hydrogène,
- l'autre des groupements $R_2$ ou $R_3$ représente un groupement Z correspondant à un atome d'hydrogène,

leurs isomères ainsi que leurs sels d 'addition à un acide ou à une base pharmaceutiquement acceptable.

**7.** Composés de formule (I) selon la revendication 5 **caractérisés en ce que** :

- l'un des groupements $R_2$ ou $R_3$ représente un groupement $W_2$ dans lequel le groupement $R_1$ représente un groupement alkyle ($C_1$-$C_6$) linéaire ou ramifié, et le groupement $R_{2a}$ représente un atome d'hydrogène,
- l'autre des groupements $R_2$ ou $R_3$ représente un groupement Z correspondant à un atome d'hydrogène,

leurs isomères ainsi que leurs sels d'addition à un acide ou à une base pharmaceutiquement acceptable.

**8.** Composés de formule (I) selon la revendication 1 **caractérisés en ce que** $R_1$ représente un groupement alkyle ($C_1$-$C_6$) linéaire ou ramifié, leurs isomères ainsi que leurs sels d'addition à un acide ou à une base pharmaceutiquement acceptable.

**9.** Composé de formule (I) selon la revendication 1 qui est le N1,N2-di[(5,10-diméthyl-5,10-dihydrodipyrido-[2,3-*b* : 3,2-*e*]pyrazin-2-yl)méthyl]-1,2-éthanediamine, ses isomères et ses sels d'addition à un acide ou à une base pharmaceutiquement acceptable.

**10.** Composé de formule (I) selon la revendication 1 qui est le N1,N3-di[(5,10-diméthyl-5,10-dihydrodipyrido-[2,3-*b* : 3,2-*e*]pyrazin-2-yl)méthyl]-1,3-propanediamine, ses isomères et ses sels d'addition à un acide ou à une base pharmaceutiquement acceptable.

**11.** Composé de formule (I) selon la revendication 1 qui est le N1,N4-di[(5,10-diméthyl)-5,10-dihydrodipyrido-[2,3-b : 3,2-c]pyrazin-2-yl)méthyl]-1,4-butanediamine, ses isomères et ses sels d'addition à un acide ou à une base pharmaceutiquement acceptable.

**12.** Composé de formule (I) selon la revendication 1 qui est le N1-[(5,10-diméthyl-5,10-dihydrodipyrido-[2,3-b : 3,2-e] pyrazin-2-yl)méthyl]-N3-(3- {[5,10-diméthyl-5,10-dihydrodipyrido[2,3-b : 3,2-e]pyrazin-2-yl)méthyl]amino}propyl) -N3-méthyl-1,3-propanediamine, ses isomères et ses sels d'addition à un acide ou à une base pharmaceutiquement acceptable.

**13.** Composé de formule (I) selon la revendication 1 qui est le N1,N7-di[(5,10-diméthyl-5,10-dihydrodipyridio-[2,3-b : 3,2-e]pyrazin-2-yl)méthyl]-1,7-heptanediamine, ses isomères et ses sels d'addition à un acide ou à une base pharmaceutiquement acceptable.

**14.** Procédé de préparation des composés de formule (I) selon la revendication 1, **caractérisé en ce que** l'on utilise comme produit de départ une 3-halogéno-2-amino-pyridine de formule (II):

L'invention s'étend également au procédé de préparation des composés de formule (I), **caractérisé en ce que** l'on utilise comme produit de départ une 3-halogéno-2-aminopyridine de formule **(II)** :

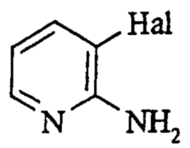

**(II)**

dans laquelle Hal représente un atome d'halogène,
composés de formule (II) que l'on fait réagir en condition basique avec un composé de formule **(III)** :

$$R'_1\text{- Hal} \quad \textbf{(III)}$$

dans laquelle Hal représente un atome d'halogène et $R'_1$ représente un groupement alkyle ($C_1$-$C_6$) linéaire ou ramifié, éventuellement substitué par un groupement alkoxy ($C_1$-$C_6$) linéaire ou ramifié, monoalkyle ou dialkylamino ($C_1$-$C_6$) linéaire ou ramifié, ou un groupement aryle,
pour conduire aux composés de formule **(IV)** :

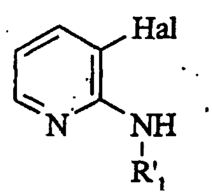

**(IV)**

dans laquelle Hal et $R'_1$ sont tels que définis précédemment,
composé de formule (IV) qui est condensé sur lui-même en présence d'une base complexe, pour conduire aux composés de formule **(I/a)**, cas particulier des composés de formule (I) :

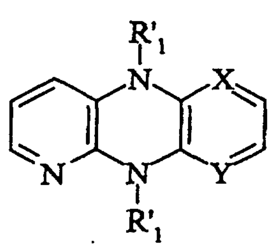

**(I/a)**

dans laquelle $R'_1$ est tel que défini précédemment et, X et Y ont la même signification que dans la formule (I), composés de formule (I/a), dans le cas particulier où $R'_1$ représente un groupement $R''_1$ consistant en un groupement alkyle ($C_1$-$C_6$) linéaire, que l'on soumet si on le désire à une réaction de métallation et dont on piège l'anion intermédiaire par un halogénure d'alkyle ($C_1$-$C_6$) dialkyldisulfure ($C_1$-$C_6$) linéaire ou ramifié, un halogénure d'alkyl ($C_1$-$C_6$) alkoxy ($C_1$-$C_6$) linéaire ou ramifié, un halogène, un aldéhyde, une cétone, un dérivé carboxylé ou alkoxycarbonylé ($C_1$-$C_6$) linéaire ou ramifié, pour conduire aux composés de formules **(I/b)** et **(I/b')**, et/ou **(I/c)**, les produits (I/b') étant des co-produits de réactions :

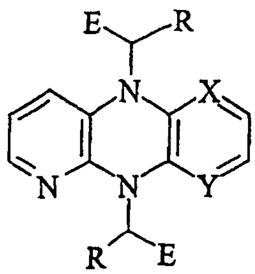
(et/ou)
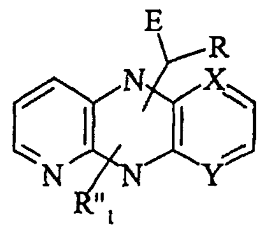
(et/ou)
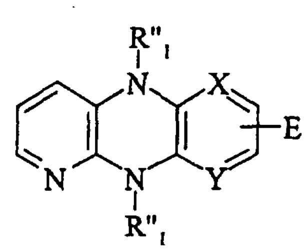

**(I/b)** **(I/b')** **(I/c)**

dans lesquelles X, Y et $R''_1$ sont tels que définis précédemment, R représente un atome d'hydrogène ou un groupement alkyle ($C_1$-$C_5$) linéaire et E représente un atome d'halogène, un groupement alkyle ($C_1$-$C_6$) linéaire ou ramifié, alkylthio ($C_1$-$C_6$) linéaire ou ramifié, formyle, alkyle ($C_1$-$C_6$) linéaire ou ramifié substitué par un groupement alkoxy ($C_1$-$C_6$) linéaire ou ramifié, ou alkyle ($C_1$-$C_6$) linéaire ou ramifié substitué par un ou plusieurs groupements, identiques ou différents, choisis parmi aryle, hydroxy, formyle, alkoxycarbonyle ($C_1$-$C_6$) linéaire ou ramifié, et carboxy,
l'ensemble des composés de formule (I/a), (I/b) et (I/b') formant les composés de formule **(I/d)** :

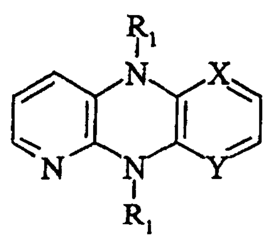
**(I/d)**

dans laquelle $R_1$, X et Y ont la même signification que dans la formule (I),
composés de formule (I/d),

- ❖ que l'on traite si on le désire par un agent de nitration, pour conduire aux composés de formule (**I/e**), cas particulier des composés de formule (I),

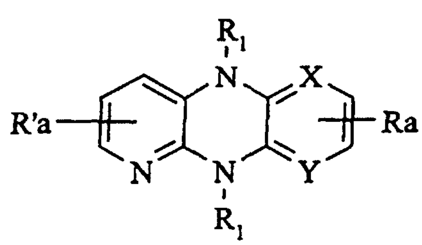
**(I/e)**

dans laquelle X, Y et $R_1$ sont tels que définis précédemment,
et Ra et R'a, identiques ou différents, représentent un atome d'hydrogène ou un groupement nitro, étant entendu que Ra et R'a ne peuvent pas représenter simultanément un atome d'hydrogène,
composés de formule (I/e) dont on réduit si on le souhaite la (les) fonction(s) nitro en fonction amine, pour conduire aux composés de formule (I/f), cas particulier des composés de formule (I) :

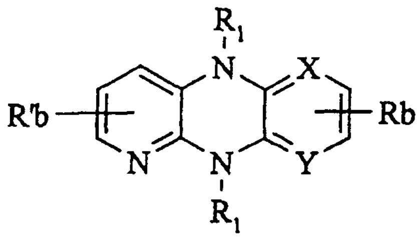

(I/f)

dans laquelle X, Y et $R_1$ sont tels que définis précédemment et Rb, R'b, identiques ou différents, représentent un atome d'hydrogène ou un groupement amino, étant entendu que Rb et R'b ne peuvent pas représenter simultanément un atome d'hydrogène,
composés de formule (I/f) que l'on traite en condition basique avec un dérivé électrophile, pour conduire aux composés de formule **(I/g)**, cas particulier des composés de formule (I) :

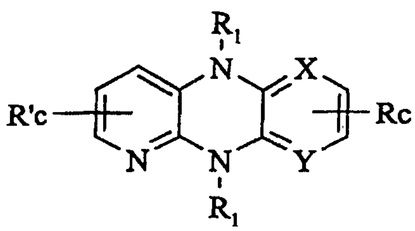

(I/g)

dans laquelle X, Y et $R_1$ sont tels que définis précédemment, et Rc, R'c, identiques ou différents, représentent un atome d'hydrogène ou un groupement amino substitué par un ou deux groupements, identiques ou différents, choisis parmi alkyle ($C_1$-$C_6$) linéaire ou ramifié, aryle, arylalkyle ($C_1$-$C_6$) linéaire ou ramifié, alkylsulfonyle ($C_1$-$C_6$) linéaire ou ramifié, arylsulfonyle, aminoalkyle ($C_1$-$C_6$) linéaire ou ramifié, monoalkylaminoalkyle ($C_1$-$C_6$) linéaire ou ramifié, dialkylaminoalkyle ($C_1$-$C_6$) linéaire ou ramifié, hydroxyalkyle ($C_1$-$C_6$) linéaire ou ramifié, et alkoxyalkyle ($C_1$-$C_6$) linéaire ou ramifié, étant entendu que Rc et R'c ne peuvent pas représenter simultanément un atome d'hydrogène,
❖ ou que l'on soumet à des conditions de réaction de formylation, pour conduire aux composés de formule (**I/h**), cas particulier des composés de formule (I) :

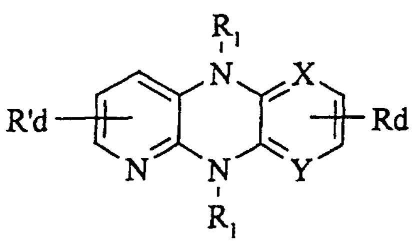

(I/h)

dans laquelle X, Y et $R_1$ sont tels que définis précédemment et Rd, R'd, identiques ou différents, représentent un groupement formyle ou un atome d'hydrogène, étant entendu que Rd et R'd ne peuvent pas représenter simultanément un atome d'hydrogène,
composés de formule (I/h)

- que l'on traite par un agent oxydant puis que l'on soumet éventuellement à l'action d'un alcool, pour conduire aux composés de formule **(I/h')**, cas particulier des composés de formule (I) :

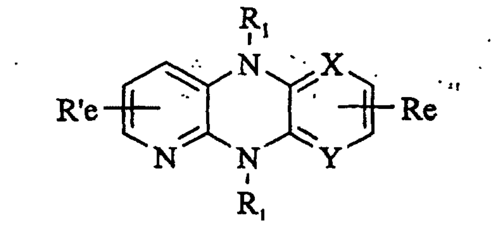

**(I/h')**

dans laquelle $R_1$, X et Y sont tels que définis précédemment et Re, R'e, identiques ou différents, représentent un atome d'hydrogène, un groupement hydroxycarbonyle ou un groupement alkoxycarbonyle ($C_1$-$C_6$) linéaire ou ramifié, étant entendu que R'e et Re ne peuvent pas représenter simultanément un atome d'hydrogène,
composés de formule (I/h') dont on transforme éventuellement au moins l'un des groupements Re et/ou R'e en chlorure d'acide, selon des conditions classiques de synthèse organique, ou que l'on fait réagir directement avec un composé amino, selon par exemple des conditions de couplage peptidique, pour conduire aux composés de formule (I/h'$_1$), cas particulier des composés de formule (I):

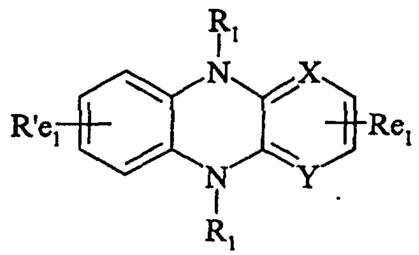

**(I/h'$_1$)**

dans laquelle $R_1$, X et Y sont tels que définis précédemment et $Re_1$, $R'e_1$, identiques ou différents, représentant un atome d'hydrogène, un groupement aminocarbonyle, monoalkylaminocarbonyle ($C_1$-$C_6$) linéaire ou ramifié, ou dialkylaminocarbonyle ($C_1$-$C_6$) linéaire ou ramifié, étant entendu que $Re_1$ et $R'e_1$ ne peuvent pas représenter simultanément un atome d'hydrogène,

- ou que l'on soumet à des conditions d'amination réductrice pour conduire aux composés de formule **(I/i)**, cas particulier des composés de formule (I) :

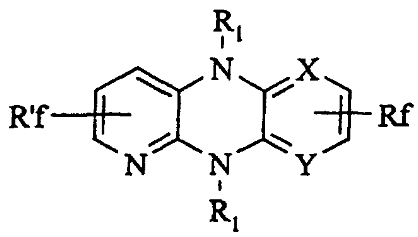

**(I/i)**

dans laquelle X, Y et $R_1$ sont tels que définis précédemment et Rf, R'f, identiques ou différents, représentent un atome d'hydrogène ou un groupement de formule $CH_2$-$NR_{10}$-$R_{20}$ dans lequel $R_{10}$ et $R_{20}$, identiques ou différents, représentent un atome d'hydrogène, un groupement alkyl ($C_1$-$C_6$) linéaire ou ramifié, aryle, arylalkyle ($C_1$-$C_6$) linéaire ou ramifié, aminoalkyle ($C_1$-$C_6$) linéaire ou ramifié, monoalkylaminoalkyle ($C_1$-$C_6$) linéaire ou ramifié, dialkylaminoalkyle ($C_1$-$C_6$) linéaire ou ramifié, hydroxyalkyle ($C_1$-$C_6$) linéaire ou ramifié, ou alkoxyalkyle ($C_1$-$C_6$) linéaire ou ramifié, étant entendu que Rf et R'f ne peuvent pas représenter simultanément un atome d'hydrogène,

- ou que l'on traite par un ylure de phosphore préparé à partir d'un composé de formule **(V)**:

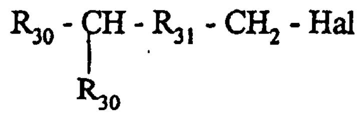

**(V)**

dans laquelle Hal représente un atome d'halogène, $R_{31}$ représente une chaîne alkylène ($C_1$-$C_4$) linéaire ou ramifiée, et $R_{30}$ représente un groupement protecteur des fonctions aldéhydes, tel qu'un acétal, pour conduire aux composés de formule **(VI)** :

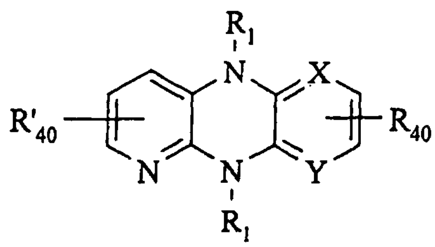

**(VI)**

dans laquelle X, Y et $R_1$ sont tels que définis précédemment et $R_{40}$ et $R'_{40}$ représentent un atome d'hydrogène ou un groupement de formule :

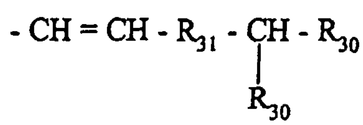

dans laquelle $R_{31}$ et $R_{30}$ sont tels que définis précédemment,
composés de formule (VI) dont on réduit la double liaison selon des conditions classiques de synthèse organique, puis dont on déprotège la fonction aldéhyde terminale, pour conduire aux composés de formule **(I/j)**, cas particulier des composés de formule (I) :

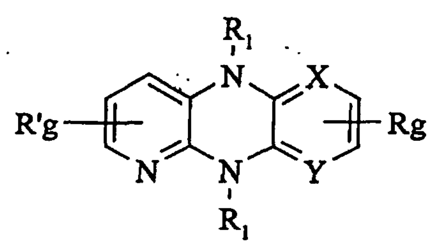

**(I/j)**

dans laquelle X, Y et $R_1$ sont tels que définis précédemment et, Rg, R'g, identiques ou différents, représentent un atome d'hydrogène ou un groupement alkyle ($C_1$-$C_6$) linéaire ou ramifiée, substitué par un groupement formyle, étant entendu que Rg et R'g ne peuvent pas représenter simultanément un atome d'hydrogène,
composés de formule (I/j) que l'on soumet à des conditions d'amination réductrice pour conduire aux composés de formule **(I/k)**, cas particulier des composés de formule (I) ;

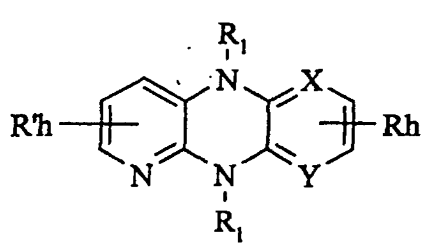

**(I/k)**

dans laquelle X, Y et $R_1$ sont tels que définis précédemment et Rh, R'h, identiques ou différents, représentent un atome d'hydrogène ou un groupement alkyle ($C_1$-$C_6$) linéaire ou ramifié, substitué par un groupement de formule $CH_2$-$NR_{10}R_{20}$ dans lequel $R_{10}$ et $R_{20}$ sont tels que définis précédemment, étant entendu que Rh et R'h ne peuvent pas représenter simultanément un atome d'hydrogène,

❖ ou que l'on soumet à un agent d'halogénation, tel que par exemple le tribromure de phényltriméthylammonium, pour conduire aux composés de formule **(I/l)**, cas particulier des composés de formule (I) :

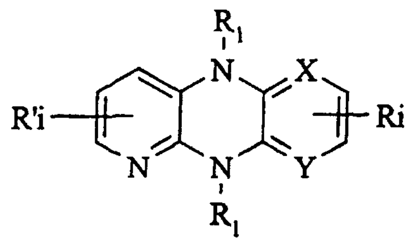

**(I/l)**

dans laquelle X, Y et $R_1$ sont tels que définis précédemment, et Ri, R'i, identiques ou différents représentent un atome d'hydrogène ou un atome d'halogène, étant entendu que Ri et R'i ne représentent pas simultanément un atome d'hydrogène,

étant entendu que les réactions précédemment décrites, effectuées à partir des composés de formule (I/d), permettent d'obtenir un ou plusieurs produits substitués soit sur un cycle pyridinyle, soit sur l'autre cycle pyridinyle soit sur les deux cycles pyridinyle, que ces différentes réactions peuvent être réalisées dans un ordre quelconque à partir des composés de formule (I/d) et/ou (I/c) et qu'ainsi selon un choix judicieux de l'homme de l'art, tant au niveau des réactifs utilisés, de l'ordre dans lequel les réactions sont effectuées, que des conditions de réaction, il est possible d'obtenir à partir des composés de formule (I/d) et (I/c) l'un quelconque des composés de formule **(I/m)**, cas particulier des composés de formule (I) :

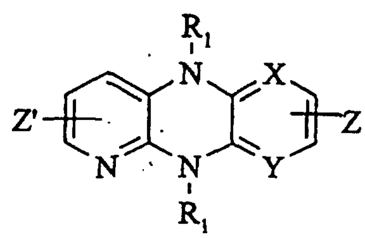

**(I/m)**

dans laquelle X, Y, $R_1$ et Z sont tels que définis dans la formule (I), et Z' représente l'une quelconque des définitions de Z,

*composés de formule (I/m)*, que l'on traite, si on le désire,

⇨ soit, dans le cas où l'un des groupements Z ou Z' représente un groupement amino ou aminoalkyle ($C_1$-$C_7$) linéaire ou ramifié, dans des conditions d'amination ou d'amination réductrice,

* *par un composé* de formule **(VII)** :

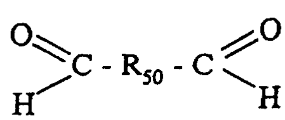

**(VII)**

dans laquelle $R_{50}$ représente une chaîne alkylène linéaire ou ramifiée, dans laquelle un ou plusieurs atomes de carbone sont remplacés éventuellement par un ou plusieurs atomes d'oxygène ou groupements $N(R_4)$ avec $R_4$ tel que défini dans la formule (I),

* ou *par un composé* de formule **(VIII)** :

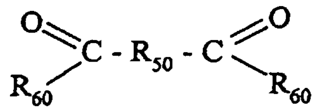

**(VIII)**

dans laquelle $R_{60}$ représente un atome de chlore ou un groupement alkoxy ($C_1$-$C_6$) linéaire ou ramifié, et $R_{50}$ est tel que défini précédemment,

⇨ soit, dans le cas où l'un des groupements Z ou Z' représente un groupement formyle ou formylalkyle ($C_1$-$C_6$) linéaire ou ramifié, dans des conditions d'amination ou d'amination réductrice,
par un composé de formule **(IX)** :

$$HR_4N - R_{70} - NR_4H \qquad \textbf{(IX)}$$

dans laquelle $R_4$ est tel que défini dans la formule (I) et $R_{70}$ représente une chaîne alkylène linéaire ou ramifié, dans laquelle un ou plusieurs atomes de carbone peuvent éventuellement être remplacés par un ou plusieurs atomes d'oxygène ou groupement $NR_4$ avec $R_4$ tel que défini précédemment,

⇨ soit, dans le cas où l'un des groupements Z ou Z' représente un groupement alkoxycarbonyle ($C_1$-$C_6$) linéaire ou ramifié, ou un chlorure d'acide, par un composé de formule (IX) tel que défini précédemment, dans des conditions classiques de couplage peptidique,

pour conduire aux composés de formule **(I/n)** ou **(I/o)**, cas particulier des composés de formule (I) :

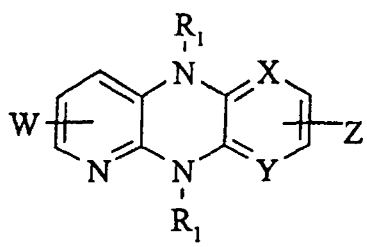

**(I/n)**

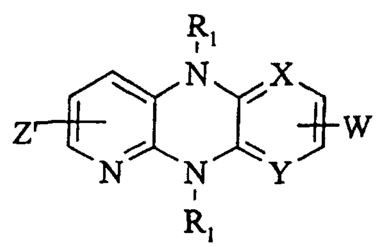

**(I/o)**

dans laquelle X, Y, $R_1$, Z et W ont la même signification que dans la formule (I), et Z' est tel que défini précédemment, étant entendu que les groupements $R_{50}$ et $R_{70}$ définis précédemment ont une longueur de chaîne telle qu'elle permette d'obtenir des composés (I/n) et (I/o) dans lesquels W comporte de 5 à 24 chaînons,
les composés (I/a) à (I/o) formant l'ensemble des composés de l'invention, que l'on purifie, le cas échéant, selon une technique classique de purification, qui peuvent, si on le désire, être séparés en leurs différents isomères selon une technique classique de séparation, et que l'on transforme, le cas échéant en leurs sels d'addition à un acide ou à une base pharmaceutiquement acceptable.

**15.** Composés de formules (α), (β) et (χ) :

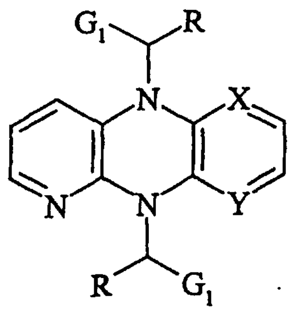

,

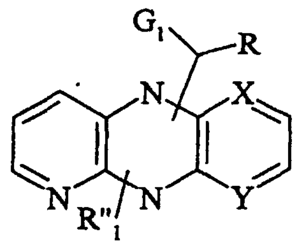

,

(α) (β)

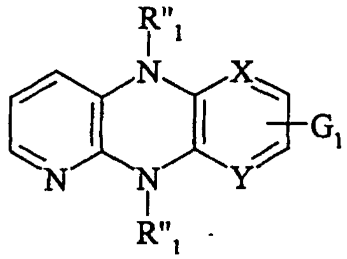

(χ)

dans laquelle :

X et Y sont tels que définis dans la formule (I),
R"$_1$ représente un groupement alkyle ($C_1$-$C_6$) linéaire,
R représente un atome d'hydrogène ou un groupement alkyle ($C_1$-$C_5$) linéaire,
$G_1$ représente un atome d'étain, de bore, ou de silicium, chacun de ces atomes étant substitué par un ou plusieurs groupements alkyle ($C_1$-$C_6$) linéaire ou ramifié, utiles en tant qu'intermédiaires de synthèse des composés de formule (I).

**16.** Procédé de préparation des composés de formules (α) et (β) et/ou (χ), utiles en tant qu'intermédiaires de synthèse des composés de formule (I), **caractérisé en ce que** l'on soumet un composé de formule (I/a), cas particulier des composés de formule (I) :

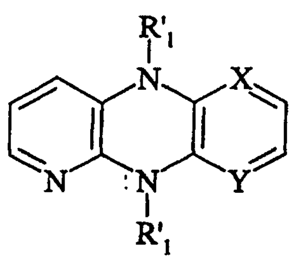

(I/a)

dans laquelle X et Y sont tels que définis dans la formule (I) et R'$_1$ représente un groupement R"$_1$ consistant en un groupement alkyle ($C_1$-$C_6$) linéaire,
à une réaction de métallation dans laquelle l'anion intermédiaire est piégé par un dérivé de l'étain, du bore, ou du silicium, permettant d'obtenir les composés de formules (α) et (β) et/ou (χ):

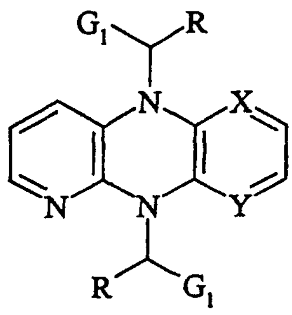

(α)

(et/ou)

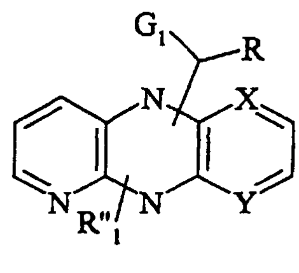

(β)

(et/ou)

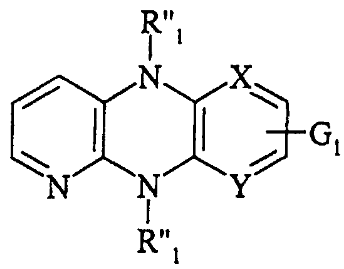

(χ)

dans lesquelles :

X, Y et $R"_1$ sont tels que définis précédemment, R représente un atome d'hydrogène ou un groupement alkyle ($C_1$-$C_5$) linéaire, et $G_1$ représente un atome d'étain, de bore, ou de silicium, chacun de ces atomes étant substitué par un ou plusieurs groupements alkyle ($C_1$-$C_6$) linéaire ou ramifié.

**17.** Compositions pharmaceutiques contenant comme principe actif au moins un composé de formule (I) selon l'une quelconque des revendications 1 à 13, seul ou en combinaison avec un ou plusieurs excipients ou véhicules inertes, non toxiques, pharmaceutiquement acceptables.

**18.** Compositions pharmaceutiques selon la revendication 17 contenant au moins un principe actif selon l'une quelconque des revendications 1 à 13, utiles dans le traitement du cancer.

**Patentansprüche**

**1.** Verbindungen der Formel (I):

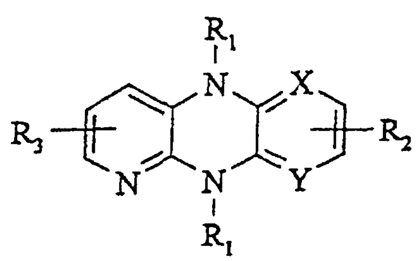

(I)

in der:

**X** ein Stickstoffatom, ein Kohlenstoffatom oder eine Gruppe CH bedeutet,
**Y** ein Stickstoffatom, wenn X ein Kohlenstoffatom oder eine Gruppe CH darstellt, oder Y ein Kohlenstoffatom oder eine Gruppe CH bedeutet, wenn X ein Stickstoffatom darstellt,

mit der Maßgabe, daß, wenn X und Y ein Kohlenstoffatom bedeuten, dieses die Gruppe $R_2$ trägt,

**$R_1$** eine geradkettige oder verzweigte ($C_1$-$C_6$)-Alkylgruppe (die gegebenenfalls durch eine oder mehrere, gleichartige oder verschiedene Gruppen ausgewählt aus Halogen, geradkettigem oder verzweigtem ($C_1$-$C_6$)-Alkylthio, geradkettigem oder verzweigtem ($C_1$-$C_6$)-Alkoxy, geradkettigem oder verzweigtem ($C_1$-$C_6$)-Monoalkyl- oder -Dialkylamino, Aryl, Hydroxy, Formyl, geradkettigem oder verzweigtem ($C_1$-$C_6$)-Alkyloxycarbonyl und Carboxy substituiert ist), mit der Maßgabe bedeutet, daß die beiden Gruppen $R_1$ entweder die Bedeutungen von identischen Gruppen oder die Bedeutungen von unterschiedlichen Gruppen besitzen,
**$R_2$ und $R_3$,** die gleichartig oder verschieden sind, unabhängig voneinander eine Gruppe Z darstellen,
<u>oder</u>
eine der Gruppen $R_2$ oder $R_3$ eine Gruppe W und die andere der Gruppen $R_2$ oder $R_3$ eine Gruppe Z bedeuten,

in welchen Gruppen Z und W:

- Z:

  * ein Wasserstoffatom,
  * ein Halogenatom,
  * eine geradkettige oder verzweigte ($C_1$-$C_6$)-Alkylgruppe, welche Alkylgruppe durch eine oder mehrere, gleichartige oder verschiedene Gruppen ausgewählt aus Hydroxy, geradkettigem oder verzweigtem ($C_1$-$C_6$)-Alkoxy, Aryl, Amino (gegebenenfalls substituiert durch eine oder zwei gleichartige oder verschiedene Gruppen ausgewählt aus geradkettigem oder verzweigtem ($C_1$-$C_6$)-Alkyl, Aryl, geradkettigem oder verzweigtem ($C_1$-$C_6$)-Arylalkyl, geradkettigem oder verzweigtem ($C_1$-$C_6$)-Aminoalkyl, geradkettigem oder verzweigtem ($C_1$-$C_6$)-Monoalkylaminoalkyl, geradkettigem oder verzweigtem ($C_1$-$C_6$)-Dialkylaminoalkyl, geradkettigem oder verzweigtem ($C_1$-$C_6$)-Hydroxyalkyl und geradkettigem oder verzweigtem ($C_1$-$C_6$)-Alkoxyalkyl), und Formyl substituiert sein kann,
  * Amino (gegebenenfalls substituiert durch eine oder zwei gleichartige oder verschiedenartige Gruppen ausgewählt aus geradkettigem oder verzweigtem ($C_1$-$C_6$)-Alkyl, Aryl, geradkettigem oder verzweigtem ($C_1$-$C_6$)-Arylalkyl, geradkettigem oder verzweigtem ($C_1$-$C_6$)-Alkylsulfonyl, Arylsulfonyl, geradkettigem oder verzweigtem ($C_1$-$C_6$)-Aminoalkyl, geradkettigem oder verzweigtem ($C_1$-$C_6$)-Monoalkylaminoalkyl, geradkettigem oder verzweigtem ($C_1$-$C_6$)-Dialkylaminoalkyl, geradkettigem oder verzweigtem ($C_1$-$C_6$)-Hydroxyalkyl und geradkettigem oder verzweigtem ($C_1$-$C_6$)-Alkoxyalkyl),
  * Nitro,
  * geradkettiges oder verzweigtes ($C_1$-$C_6$)-Alkylthio,
  * Formyl,
  * Hydroxycarbonyl,
  * geradkettiges oder verzweigtes ($C_1$-$C_6$)-Alkoxycarbonyl,
  * Aminocarbonyl, geradkettiges oder verzweigtes ($C_1$-$C_6$)-Monoalkylaminocarbonyl oder eine geradkettige oder verzweigte ($C_1$-$C_6$)-Dialkylaminocarbonylgruppe bedeutet,

- W eine geradkettige oder verzweigte ($C_5$-$C_{24}$)-Alkylenkette darstellt, in der eines oder mehrere Kohlenstoffatome unabhängig voneinander gegebenenfalls durch eine oder mehrere gleichartige oder verschiedene Gruppen ersetzt ist ausgewählt aus Sauerstoffatomen, Imingruppen, -N($R_4$)-CO-, -CO-N($R_4$)- und N($R_4$), worin $R_4$ ein Wasserstoffatom, eine geradkettige oder verzweigte ($C_1$-$C_6$)-Alkylgruppe, Arylgruppe oder geradkettige oder verzweigte ($C_1$-$C_6$)-Arylalkylgruppe bedeutet, welche Alkylenkette in ihrer Endposition durch eine der folgenden Gruppen substituiert ist:

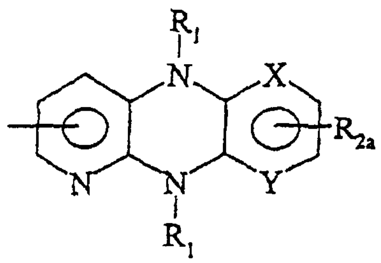
,
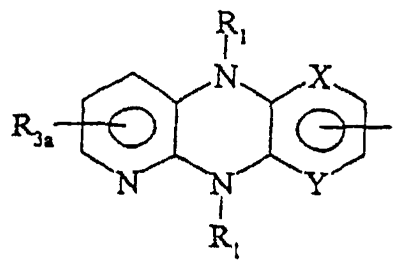

in denen X, Y und $R_1$ die oben angegebenen Bedeutungen besitzen und $R_{2a}$ und $R_{3a}$ eine Gruppe Z bedeuten, wie sie oben definiert worden ist,

deren Isomere sowie deren Additionssalze mit einer pharmazeutisch annehmbaren Säure oder Base,
wobei es sich versteht, daß unter einer Arylgruppe eine Phenyl-, Naphthyl-, Tetrahydronaphthyl-, Dihydronaphthyl-, Indenyl- oder Indanylgruppe zu verstehen ist, wobei jede dieser Gruppen gegebenenfalls in identischer oder unterschiedlicher Weise substituiert ist durch ein oder mehrere Halogenatome, Hydroxygruppen, Cyanogruppen, Nitrogruppen, geradkettige oder verzweigte ($C_1$-$C_6$)-Alkylgruppen, geradkettige oder verzweigte ($C_1$-$C_6$)-Trihalogenalkylgruppen, geradkettige oder verzweigte ($C_1$-$C_6$)-Alkoxygruppen, geradkettige oder verzweigte ($C_1$-$C_6$) -Acylgruppen, Carboxygruppen, geradkettige oder verzweigte ($C_1$-$C_6$)-Alkoxycarbonylgruppen oder Aminogruppen (die gegebenenfalls durch eine oder zwei gleichartige oder verschiedene Gruppen ausgewählt aus geradkettigem oder verzweigtem ($C_1$-$C_6$)-Alkyl, Aryl und geradkettigem oder verzweigtem ($C_1$-$C_6$)-Arylalkyl substituiert ist).

**2.** Verbindungen der Formel (I) nach Anspruch 1, **dadurch gekennzeichnet, daß** $R_2$ und $R_3$, die gleichartig oder verschieden sind, unabhängig voneinander eine Gruppe Z bedeuten, deren Isomere sowie deren Additionssalze mit einer pharmazeutisch annehmbaren Säure oder Base.

**3.** Verbindungen der Formel (I) nach Anspruch 1, **dadurch gekennzeichnet, daß** eine der Gruppen $R_2$ oder $R_3$ eine Gruppe Z und die andere der Gruppen $R_2$ oder $R_3$ eine Gruppe W bedeuten, deren Isomere sowie deren Additionssalze mit einer pharmazeutisch annehmbaren Säure oder Base.

**4.** Verbindungen der Formel (I) nach einem der Ansprüche 1 oder 3, **dadurch gekennzeichnet, daß** eine der Gruppen $R_2$ oder $R_3$ eine Gruppe Z und die andere der Gruppen R2 oder $R_3$ eine Gruppe $W_1$ bedeuten, welche Gruppe $W_1$ eine geradkettige ($C_6$-$C_{12}$)-Alkylenkette darstellt, in der zwei oder drei Kohlenstoffatome durch zwei oder drei gleichartige oder verschiedene Gruppen $N(R_4)$ ersetzt sind, worin $R_4$ ein Wasserstoffatom oder eine geradkettige oder verzweigte ($C_1$-$C_6$)-Alkylgruppe darstellt, welche Alkylenkette in ihrer Endposition durch eine der beiden folgenden Gruppen substituiert ist:

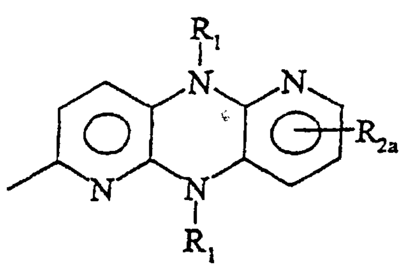
,
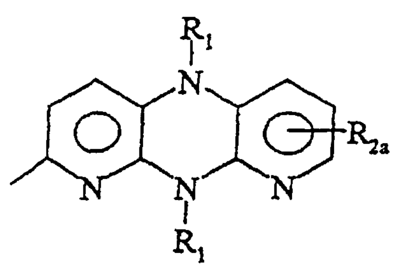

in denen $R_1$ die bezüglich der allgemeinen Formel (I) angegebenen Bedeutungen besitzt und $R_{2a}$ eine Gruppe Z darstellt, deren Isomere sowie deren Additionssalze mit einer pharmazeutisch annehmbaren Säure oder Base.

**5.** Verbindungen der Formel (I) nach einem der Ansprüche 1 oder 3, **dadurch gekennzeichnet, daß** eine der Gruppen R2 oder $R_3$ eine Gruppe Z und die andere der Gruppen $R_2$ oder $R_3$ eine Gruppe $W_2$ der Formel bedeuten:

$$- CO-N(R_4)-W'_2-N(R_4)-CO-G$$

in der:

- jede der Gruppen $R_4$, die gleichartig oder verschieden sind, ein Wasserstoffatom oder eine geradkettige oder verzweigte ($C_1$-$C_6$)-Alkylgruppe darstellt,
- W'2 eine geradkettige ($C_3$-$C_{12}$)-Alkylenkette darstellt, in der eines, zwei oder drei Kohlenstoffatome gegebenenfalls durch eine, zwei oder drei gleichartige oder verschiedene Gruppen N($R_4$) ersetzt sind, worin $R_4$ ein Wasserstoffatom oder eine geradkettige oder verzweigte ($C_1$-$C_6$)-Alkylgruppe darstellt,
- und G eine der folgenden beiden Gruppen bedeutet:

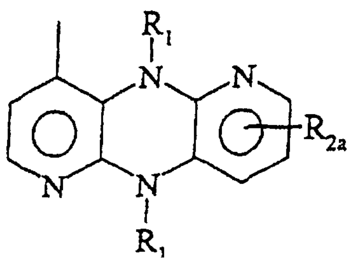
,
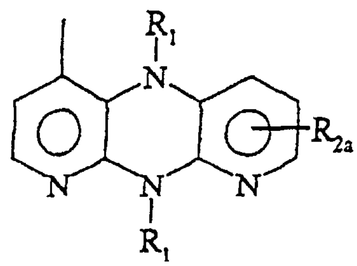

in denen $R_1$ die bezüglich der allgemeinen Formel (I) angegebenen Bedeutungen besitzt und $R_{2a}$ eine Gruppe Z darstellt,

deren Isomere sowie deren Additionssalze mit einer pharmazeutisch annehmbaren Säure oder Base.

**6.** Verbindungen der Formel (I) nach Anspruch 4, **dadurch gekennzeichnet, daß**:

- eine der Gruppen $R_2$ oder $R_3$ eine Gruppe $W_1$, worin die Gruppe $R_1$ eine geradkettige oder verzweigte ($C_1$-$C_6$) -Alkylgruppe darstellt, und die Gruppe $R_{2a}$ ein Wasserstoffatom bedeuten,
- und die andere der Gruppen $R_2$ oder $R_3$ eine Gruppe Z darstellt, die einem Wasserstoffatom entspricht,

deren Isomere sowie deren Additionssalze mit einer pharmazeutisch annehmbaren Säure oder Base.

**7.** Verbindungen der Formel (I) nach Anspruch 5, **dadurch gekennzeichnet, daß**:

- eine der Gruppen $R_2$ oder $R_3$ eine Gruppe $W_2$ darstellt, in der die Gruppe $R_1$ eine geradkettige oder verzweigte ($C_1$-$C_6$)-Alkylgruppe darstellt, und die Gruppe $R_{2a}$ ein Wasserstoffatom bedeutet, und
- die andere der Gruppen $R_2$ oder $R_3$ eine Gruppe Z darstellt, die einem Wasserstoffatom entspricht,

deren Isomere sowie deren Additionssalze mit einer pharmazeutisch annehmbarten Säure oder Base.

**8.** Verbindungen der Formel (I) nach Anspruch 1, **dadurch gekennzeichnet, daß** $R_1$ eine geradkettige oder verzweigte ($C_1$-$C_6$)-Alkylgruppe darstellt, deren Isomeren sowie deren Additionssalze mit einer pharmazeutisch annehmbaren Säure oder Base.

**9.** Verbindung der Formel (I) nach Anspruch 1, nämlich N1,N2-Di-[(5,10-dimethyl-5,10-dihydrodipyrido-(2,3-*b*:3,2-*e*] pyrazin-2-yl)-methyl]-1,2-ethandiamin, dessen Isomere und dessen Additionssalze mit einer pharmazeutisch annehmbaren Säure oder Base.

**10.** Verbindung der Formel (I) nach Anspruch 1, nämlich N1,N3-Di-[(5,10-dimethyl-5,10-dihydrodipyrido-[2,3-*b*:3,2-*e*] pyrazin-2-yl)-methyl]-1,3-propandiamin, dessen Isomere und dessen Additionssalze mit einer pharmazeutisch annehmbaren Säure oder Base.

**11.** Verbindung der Formel (I) nach Anspruch 1, nämlich N1,N4-Di-[(5,10-dimethyl-5,10-dihydrodipyrido-[2,3-b:3,2-c] pyrazin-2-yl)-methyl]-1,4-butandiamin, dessen Isomere und dessen Additionssalze mit einer pharmazeutisch annehmbaren Säure oder Base.

**12.** Verbindung der Formel (I) nach Anspruch 1, nämlich N1-[(5,10-Dimethyl-5,10-dihydrodipyrido-[2,3-b:3.2-e]pyrazin-2-yl)-methyl]-N3-(3-{[5,10-dimethyl-5,10-dthydrodipyrido[2,3-b:3,2-e]pyrazin-2-yl)-methyl]-amino}-propyl)-N3-methyl1,3-propandiamin, dessen Isomere und dessen Additionssalze mit einer pharmazeutisch annehmbaren Säure oder Base.

**13.** Verbindung der Formel (I) nach Anspruch 1, nämlich N1,N7-Di-[(5,10-dimethyl-5,10-dihydrodipyrido-[2,3-b:3,2-e] pyrazin-2-yl)-methyl]-1,7-heptandiamin, dessen Isomere und dessen Additionssalze mit einer pharmazeutisch annehmbaren Säure oder Base.

**14.** Verfahren zur Herstellung der Verbindungen der Formel (I) nach Anspruch 1, **dadurch gekennzeichnet, daß** man als Ausgangsprodukt ein 3-Halogen-2-amino-pyridin der Formel (**II**) verwendet:

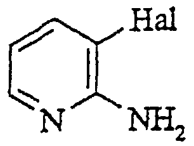

**(II)**

in der Hal ein Halogenatom darstellt,
welche Verbindungen der Formel (II) man unter basischen Bedingungen mit einer Verbindung der Formel (**III**) umsetzt:

$$R'_1 - Hal \qquad (\textbf{III})$$

in der Hal ein Halogenatom und $R'_1$ eine geradkettige oder verzweigte ($C_1$-$C_6$)-Alkylgruppe, die gegebenenfalls durch eine geradkettige oder verzweigte ($C_1$-$C_6$)-Alkoxygruppe, geradkettige oder verzweigte ($C_1$-$C_6$)-Monoalkylgruppe, geradkettige oder verzweigte ($C_1$-$C_6$)-Dialkylaminogruppe substituiert ist, oder eine Arylgruppe bedeuten,
zur Bildung der Verbindungen der Formel **(IV)**:

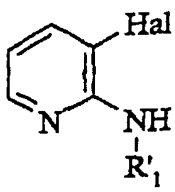

**(IV)**

in der Hal und $R'_1$ die oben angegebenen Bedeutungen besitzen,
welche Verbindung der Formel (IV) mit sich selbst in Gegenwart einer komplexen Base kondensiert wird,
zur Bildung der Verbindungen der Formel (**I/a**), einem Sonderfall der Verbindungen der Formel (I):

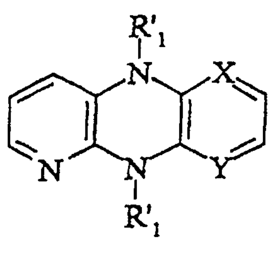

**(I/a)**

in der $R'_1$ die oben angegebenen Bedeutungen besitzen und X und Y die bezüglich der Formel (I) angegebenen Bedeutungen besitzen,
welche Verbindungen der Formel (I/a) man in dem besonderen Fall, da $R'_1$ eine Gruppe $R''_1$ darstellt, die aus einer geradkettigen ($C_1$-$C_6$)-Alkylgruppe besteht, gewünschtenfalls einer Metallierungsreaktion unterwirft und das erhaltene Zwischenproduktanion mit einem geradkettigen oder verzweigten ($C_1$-$C_6$)-Dialkyldisulfid-($C_1$-$C_6$)-alkylhalogenid, einem geradkettigen oder verzweigten ($C_1$-$C_6$)-Alkoxy-($C_1$-$C_6$)-alkylhalogenid, einem Halogen, einem Aldehyd, einem Keton, einem Carboxylderivat oder einem geradkettigen oder verzweigten ($C_1$-$C_6$)-Alkoxycarbonylderivat kondensiert zur Bildung der Verbindungen der Formel (**I/b**) und (**I/b'**) und/oder (**I/c**), wobei die Produkte ((I/b') Reaktions-Nebenprodukte sind:

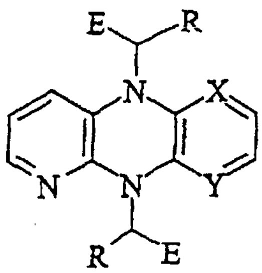

(und/oder)

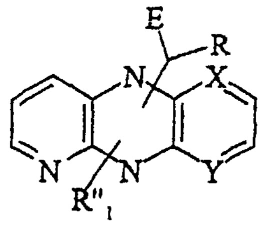

(und/oder)

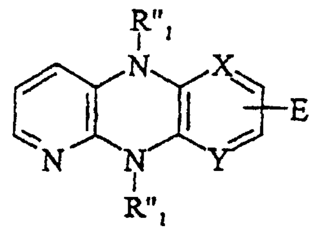

**(I/b)** **(I/b')** **(I/c)**

worin X, Y und $R''_1$ die oben angegebenen Bedeutungen besitzen, R ein Wasserstoffatom oder eine geradkettige oder verzweigte ($C_1$-$C_5$)-Alkylgruppe darstellt und E ein Halogenatom, eine geradkettige oder verzweigte ($C_1$-$C_6$)-Alkylgruppe, geradkettige oder verzweigte ($C_1$-$C_6$)-Alkylthiogruppe, Formylgruppe, geradkettige oder verzweigte ($C_1$-$C_6$)-Alkylgruppe, die durch eine geradkettige oder verzweigte ($C_1$-$C_6$)-Alkoxygruppe oder eine geradkettige oder verzweigte ($C_1$-$C_6$)-Alkylgruppe, die durch eine oder mehrere gleichartige oder verschiedene Gruppen substituiert ist, ausgewählt aus Aryl, Hydroxy, Formyl, geradkettigem oder verzweigtem ($C_1$-$C_6$)-Alkoxycarbonyl und Carboxy, substituiert ist, bedeutet,
wobei die Gesamtheit der Verbindungen der Formeln (I/a), (I/b) und (I/b') die Verbindungen der Formel (**I/d**) bilden:

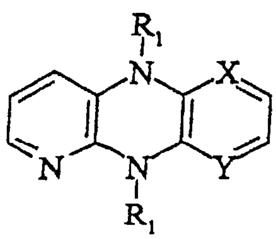

**(I/d)**

in der $R_1$, X und Y die bezüglich der Formel (I) angegebenen Bedeutungen besitzen,
welche Verbindungen der Formel (I/d)

- man gewünschtenfalls mit einem Nitrierungsmittel behandelt zur Bildung der Verbindungen der Formel (**I/e**), einem Sonderfall der Verbindungen der Formel (I):

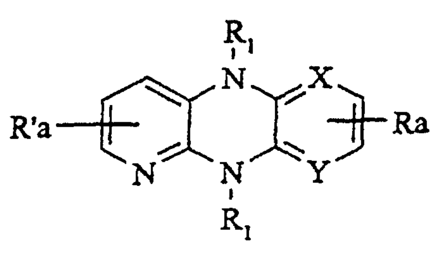

**(I/e)**

in der X, Y und $R_1$ die oben angegebenen Bedeutungen besitzen,
und Ra und R'a, die gleichartig oder verschieden sind. Wasserstoffatome oder Nitrogruppen mit der Maßgabe bedeuten, daß Ra und R'a nicht gleichzeitig Wasserstoffatom darstellen,
bei welchen Verbindungen der Formel (I/e) man gewünschtenfalls die Nitrofunktion(en) zu der (den) Aminfunktion(en) reduziert zur Bildung der Verbindungen der Formel (**I/f**), einem Sonderfall der Verbindungen der Formel (I):

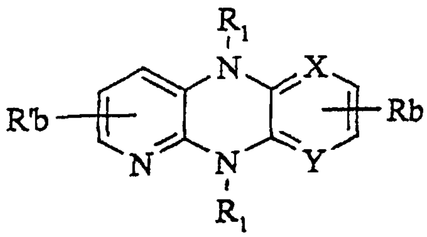

(I/f)

in der X, Y und $R_1$ die oben angegebenen Bedeutungen besitzen und Rb und R'b, die gleichartig oder verschieden sind, Wasserstoffatome oder Aminogruppen mit der Maßgabe bedeuten, daß Rb und R'b nicht gleichzeitig Wasserstoffatome darstellen.
welche Verbindungen der Formel (I/f) man unter basischen Bedingungen mit einem elektrophilen Derivat behandelt zur Bildung der Verbindungen der Formel (**I/g**), einem Sonderfall der Verbindungen der Formel (I):

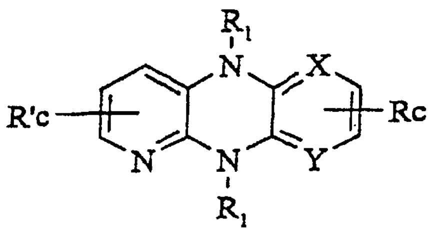

(I/g)

in der X, Y und $R_1$ die oben angegebenen Bedeutungen besitzen und Rc und R'c, die gleichartig oder verschieden sind, Wasserstoffatome oder Aminogruppen bedeuten, die durch eine oder zwei gleichartige oder verschiedene Gruppen substituiert ist ausgewählt aus geradkettigem oder verzweigtem ($C_1$-$C_6$)-Alkyl, Aryl, geradkettigem oder verzweigtem ($C_1$-$C_6$)-Arylalkyl, geradkettigem oder verzweigtem ($C_1$-$C_6$)-Alkylsulfonyl, Arylsulfonyl, geradkettigem oder verzweigtem ($C_1$-$C_6$)-Aminoalkyl, geradkettigem oder verzweigtem ($C_1$-$C_6$) -Monoalkylaminoalkyl, geradkettigem oder verzweigtem ($C_1$-$C_6$)-Dialkylamlnoalkyl, geradkettigem oder verzweigtem ($C_1$-$C_6$)-Hydroxyalkyl und geradkettigem oder verzweigtem ($C_1$-$C_6$)-Alkoxyalkyl, mit der Maßgabe, daß Rc und R'c nicht gleichzeitig Wasserstoffatome darstellen,
❖ oder man den Bedingungen der Formylierungsreaktion unterwirft zur Bildung der Verbindungen der Formel (**I/h**), einem Sonderfall der Verbindungen der Formel (I):

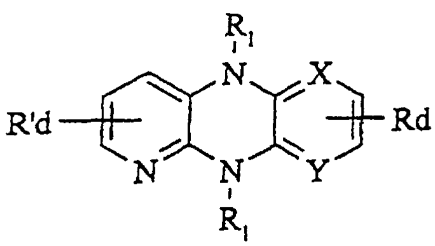

(I/h)

in der X, Y und $R_1$ die oben angegebenen Bedeutungen besitzen und Rd und R'd, die gleichartig oder verschieden sind, Formylgruppen oder Wasserstoffatome mit der Maßgabe bedeuten, daß Rd und R'd nicht gleichzeitig Wasserstoffatome darstellen,
welche Verbindungen der Formel (I/h)

- man mit einem Oxidationsmittel behandelt und dann gegebenenfalls der Einwirkung eines Alkohols unterwirft zur Bildung der Verbindungen der Formel (**I/h'**), einem Sonderfall der Verbindungen der Formel (I):

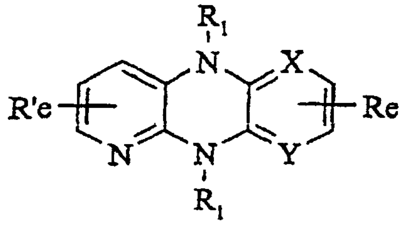

(I/h')

in der $R_1$, X und Y die oben angegebenen Bedeutungen besitzen und Re und R'e, die gleichartig oder verschieden sind, Wasserstoffatome, Hydroxycarbonylgruppen oder geradkettige oder verzweigte ($C_1$-$C_6$)-Alkoxycarbonylgruppen mit der Maßgabe bedeuten, daß R'e und Re nicht gleichzeitig Wasserstoffatome darstellen,

von welchen Verbindungen der Formel (I/h') man gegebenenfalls mindestens eine der Gruppen Re und/oder R'e unter klassischen Bedingungen der organischen Synthese in das Säurechlorid umwandelt, welches man direkt mit einer Aminogruppe umsetzt, beispielsweise unter Anwendung von Peptidkupplungsbedingungen, zur Bildung der Verbindungen der Formel (**I/h'$_1$**), einem Sonderfall der Verbindungen der Formel (I):

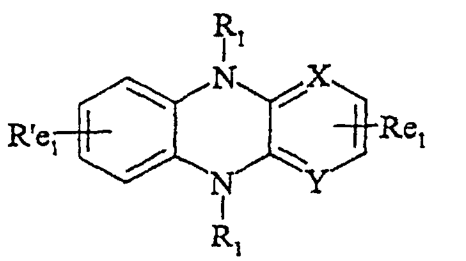

(I/h'$_1$)

in der $R_1$, X und Y die oben angegebenen Bedeutungen besitzen und $Re_1$ und $R'e_1$, die gleichartig oder verschieden sind, Wasserstoffatome, Aminocarbonylgruppen, geradkettige oder verzweigte ($C_1$-$C_6$)-Monoalkylaminocarbonylgruppen oder geradkettige oder verzweigte ($C_1$-$C_6$)-Dialkylaminocarbonylgruppen mit der Maßgabe bedeuten, daß $Re_1$ und $R'e_1$ nicht gleichzeitig Wasserstoffatome darstellen,

- oder man den Bedingungen der reduktiven Aminierung unterwirft zur Bildung der Verbindungen der Formel (**I/i**), einem Sonderfall der Verbindungen der Formel (I):

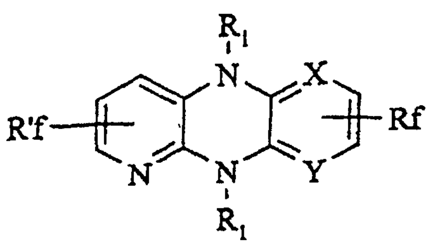

(I/i)

in der X, Y und $R_1$ die oben angegebenen Bedeutungen besitzen und Rf und R'f, die gleichartig oder verschieden sind, Wasserstoffatome oder Gruppen der Formel $CH_2$-$NR_{10}$-$R_{20}$ darstellen, worin $R_{10}$ und $R_{20}$, die gleichartig oder verschieden sind, Wasserstoffatome, geradkettige oder verzweigte ($C_1$-$C_6$)-Alkylgruppen, Arylgruppen, geradkettige oder verzweigte ($C_1$-$C_6$)-Arylalkylgruppen, geradkettige oder verzweigte ($C_1$-$C_6$)-Aminoalkylgruppen, geradkettige oder verzweigte ($C_1$-$C_6$)-Monoalkylaminoalkylgruppen, geradkettige oder verzweigte ($C_1$-$C_6$)-Dialkylaminoalkylgruppen, geradkettige oder verzweigte ($C_1$-$C_6$)-Hydroxyalkylgruppen oder geradkettige oder verzweigte ($C_1$-$C_6$)-Alkoxyalkylgruppen mit der Maßgabe bedeuten, daß Rf und R'f nicht gleichzeitig Wasserstoffatome darstellen,

- oder man mit einem Phosphorylid behandelt, welches man ausgehend von einer Verbindung der Formel (**V**) hergestellt hat:

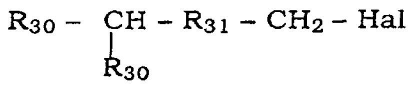 (V)

in der Hal ein Halogenatom, $R_{31}$ eine geradkettige oder verzweigte ($C_1$-$C_4$)-Alkylenkette und $R_{30}$ eine Schutzgruppe für die Aldehydfunktionen, beispielsweise eine Acetalgruppe, bedeuten, zur Bildung der Verbindungen der Formel (**VI**):

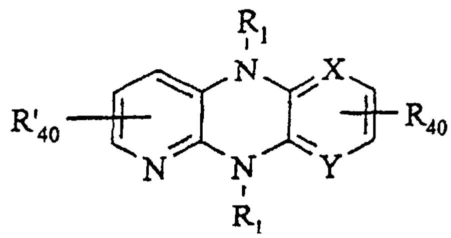 (VI)

in der X, Y und $R_1$ die oben angegebenen Bedeutungen besitzen und $R_{40}$ und $R'_{40}$ Wasserstoffatome oder Gruppen der Formel bedeuten:

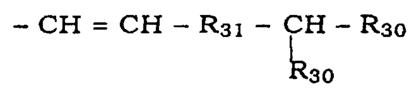

in der $R_{31}$ und $R_{30}$ die oben angegebenen Bedeutungen besitzen, bei welchen Verbindungen der Formel (VI) man die Doppelbindung unter Anwendung klassischer Bedingungen der organischen Synthese reduziert und dann die Schutzgruppe der endständigen Aldehydfunktion abspaltet zur Bildung der Verbindungen der Formel (**I/j**), einem Sonderfall der Verbindungen der Formel (I):

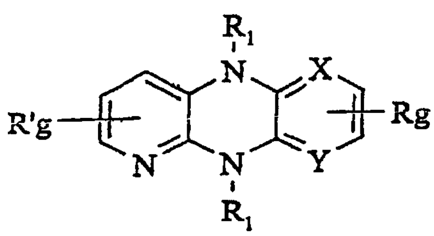 (I/j)

in der X, Y und $R_1$ die oben angegebenen Bedeutungen besitzen und Rg und R'g, die gleichartig oder verschieden sind, Wasserstoffatome oder geradkettige oder verzweigte ($C_1$-$C_6$)-Alkylgruppen, die durch eine Formylgruppe substituiert sind. mit der Maßgabe bedeuten, daß Rg und R'g nicht gleichzeitig Wasserstoffatome darstellen, welche Verbindungen der Formel (I/j) man den Bedingungen der reduktiven Aminierung unterwirft zur Bildung der Verbindungen der Formel (**I/k**), einem Sonderfall der Verbindungen der Formel (I):

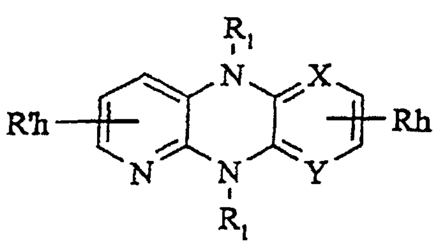 (I/k)

in der X, Y und $R_1$ die oben angegebenen Bedeutungen besitzen und Rh und R'h, die gleichartig oder

verschieden sind, Wasserstoffatome oder geradkettige oder verzweigte ($C_1$-$C_6$)-Alkylgruppen, die durch eine Gruppe der Formel $CH_2$-$NR_{10}R_{20}$ substituiert sind, in der $R_{10}$ und $R_{20}$ die oben angegebenen Bedeutungen besitzen, mit der Maßgabe bedeuten, daß Rh und R'h nicht gleichzeitig Wasserstoffatome darstellen,

❖ oder man der Einwirkung eines Halogenierungsmittels, wie beispielsweise Phenyltrimethylammoniumtribromid, unterwirft zur Bildung der Verbindungen der Formel (**I/l**), einem Sonderfall der Verbindungen der Formel (I):

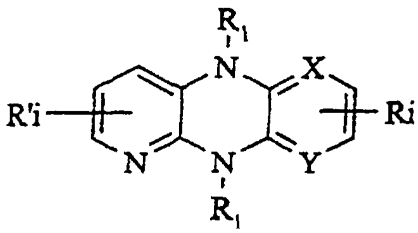

(I/l)

In der X, Y und $R_1$ die oben angegebenen Bedeutungen besitzen und Ri und R'i, die gleichartig oder verschieden sind, Wasserstoffatome oder Halogenatome mit der Maßgabe bedeuten, daß Ri und R'i nicht gleichzeitig Wasserstoffatome darstellen,
wobei es sich versteht, daß die oben beschriebenen Reaktionen, die ausgehend von den Verbindungen der Formel (I/d) durchgeführt werden, die Bildung eines oder mehrerer Produkte ermöglichen, die entweder an dem einen Pyridinylring oder an dem anderen Pyridinylring oder an beiden Pyridinylringen substituiert sind, wobei diese verschiedenen Reaktionen ausgehend von den Verbindungen der Formel (I/d) und/oder (I/c) in beliebiger Reihenfolge durchgeführt werden können, so daß man gemäß der fachmännischen Auswahl sowohl im Hinblick auf die verwendeten Reaktionsteilnehmer, die Reihenfolge in der die Reaktionen durchgeführt werden und die Reaktionsbedingungen, ausgehend von den Verbindungen der Formel (I/d) und (I/c) beliebige Verbindungen der Formel (**I/m**), einem Sonderfall der Verbindungen der Formel (I) erhalten kann:

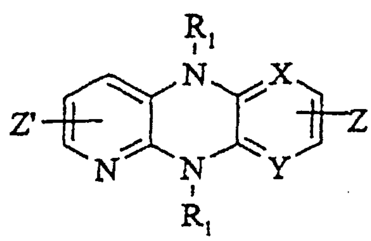

(I/m)

in der X, Y, $R_1$ und Z die bezüglich der Formel (I) angegebenen Bedeutungen besitzen und Z' eine der Bedeutungen von Z besitzt, *welche Verbindungen der Formel (I/m)* man gewünschtenfalls
⇨ entweder dann, wenn eine der Gruppen Z oder Z' eine Aminogruppe oder eine geradkettige oder verzweigte ($C_1$-$C_7$)-Aminoalkylgruppe darstellt, man unter den Bedingungen der Aminierung oder der reduktiven Aminierung

* *mit einer Verbindung* der Formel (**VII**) behandelt:

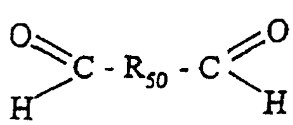

(VII)

in der $R_{50}$ eine geradkettige oder verzweigte Alkylenkette darstellt, in der eines oder mehrere Kohlenstoffatome gegebenenfalls durch eines oder mehrere Sauerstoffatome oder Gruppen $N(R_4)$, worin $R_4$ die bezüglich der Formel (I) angegebenen Bedeutungen besitzt, ersetzt sind,

* oder *mit einer Verbindung* der Formel (**VIII**):

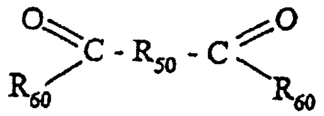

(VIII)

in der $R_{60}$ ein Chloratom oder eine geradkettige oder verzweigte ($C_1$-$C_6$)-Alkoxygruppe darstellt und $R_{50}$ die oben angegebenen Bedeutungen besitzt, behandelt,

⇨ oder dann, wenn eine der Gruppen Z oder Z' eine Formylgruppe oder eine geradkettige oder verzweigte ($C_1$-$C_6$)-Formylalkylgruppe darstellt, unter den Bedingungen der Aminierung oder der reduktiven Aminierung mit einer Verbindung der Formel (**IX**) behandelt:

$$HR_4N - R_{70} - NR_4H \qquad (\mathbf{IX})$$

in der $R_4$ die bezüglich der Formel (I) angegebenen Bedeutungen besitzt und $R_{70}$ eine geradkettige oder verzweigte Alkylenkette darstellt, in der eines oder mehrere Kohlenstoffatome gegebenenfalls durch eines oder mehrere Sauerstoffatome oder Gruppen $NR_4$, worin $R_4$ die oben angegebenen Bedeutungen besitzt, ersetzt sein können,
⇨ oder dann, wenn eine der Gruppen Z oder Z' eine geradkettige oder verzweigte ($C_1$-$C_6$)-Alkoxycarbonylgruppe oder ein Säurechlorid bedeuten, mit einer Verbindung der Formel (IX), wie sie oben definiert worden ist, unter den klassischen Peptidkupplungsbedingungen behandelt,
zur Bildung der Verbindungen der Formel (**I/n**) oder (**I/o**), einem Sonderfall der Verbindungen der Formel (I):

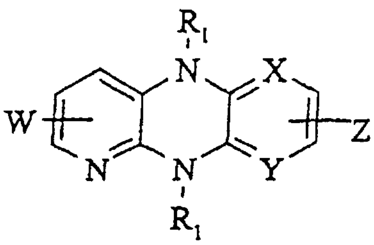

(I/n)

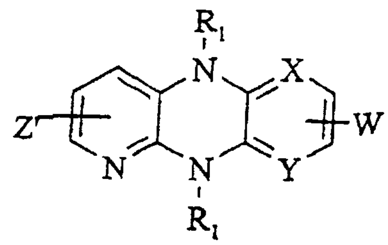

(I/o)

in der X, Y, R1, Z und W die bezüglich der Formel (I) angegebenen Bedeutungen besitzen und Z' die oben angegebenen Bedeutungen aufweist,

wobei es sich versteht, daß die oben definierten Gruppen $R_{50}$ und $R_{70}$ eine solche Kettenlänge besitzen, daß man Verbindungen der Formel (I/n) und (I/o) erhält, in denen W 5 bis 24 Kettenglieder aufweist, welche Verbindungen der Formeln (I/a) bis (I/o) die Gesamtheit der Verbindungen der Erfindung bilden, welche man gegebenenfalls mit Hilfe einer klassischen Reinigungsmethode reinigt, welche gewünschtenfalls mit Hilfe einer klassischen Trennmethode in ihre verschiedenen Isomeren getrennt werden können und welche man gegebenenfalls in ihre Additionssalze mit einer pharmazeutisch annehmbaren Säure oder Base umwandeln kann.

**15.** Verbindungen der Formeln ($\alpha$), ($\beta$) und ($\chi$):

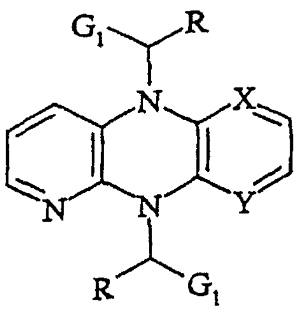

,

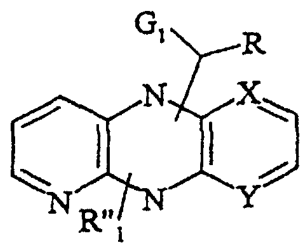

,

(α)

(β)

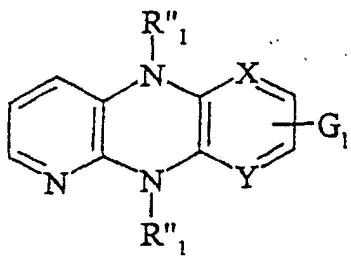

(χ)

in denen:

X und Y die bezüglich der Formel (I) angegebenen Bedeutungen besitzen,
R"$_1$ eine geradkettige ($C_1$-$C_6$)-Alkylgruppe bedeutet,
R ein Wasserstoffatom oder eine geradkettige ($C_1$-$C_5$)-Alkylgruppe darstellt und
$G_1$ ein Zinn-, Bor- oder Siliciumatom bedeutet, wobei jedes dieser Atome durch eine oder mehrere geradkettige oder verzweigte ($C_1$-$C_6$)-Alkylgruppen substituiert ist,

als Zwischenprodukte für die Synthese der Verbindungen der Formel (I).

**16.** Verfahren zur Herstellung der Verbindungen der Formeln (α) und (β) und/oder (χ), die als Zwischenprodukte für die Synthese der Verbindungen der Formel (I) nützlich sind, **dadurch gekennzeichnet, daß** man eine Verbindung der Formel (I/a), einem Sonderfall der Verbindungen der Formel (I):

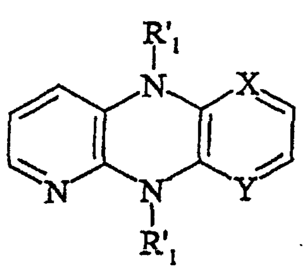

(I/a)

in der X und Y die bezüglich der Formel (I) angegebenen Bedeutungen besitzen und R'$_1$ für eine Gruppe R"$_1$ steht, die aus einer geradkettigen ($C_1$-$C_6$)-Alkylgruppe besteht,
einer Metallierungsreaktion unterwirft, dessen Zwischenproduktanion man mit einem Zinn-, Bor- oder Siliciumderivat kuppelt, welches die Bildung der Verbindungen der Formel (α) und (β) und/oder (χ) ermöglicht:

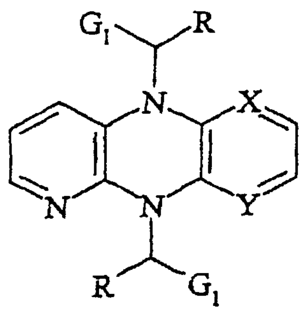

(und/oder)

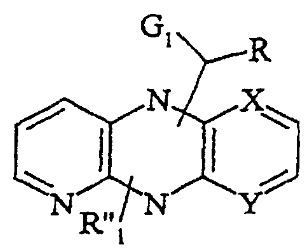

(α) (β)

(und/oder)

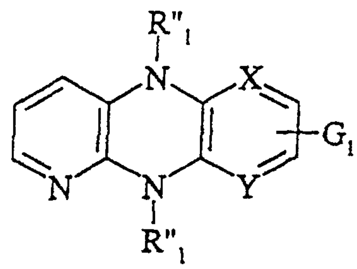

(χ)

in denen:

X, Y und $R''_1$ die oben angegebenen Bedeutungen besitzen, R ein Wasserstoffatom oder eine geradkettige oder verzweigte ($C_1$-$C_5$)-Alkylgruppe darstellt und $G_1$ ein Zinn-, Bor- oder Siliciumatom darstellt, wobei jedes dieser Atome durch eine oder mehrere geradkettige oder verzweigte ($C_1$-$C_6$)-Alkylgruppen substituiert ist.

**17.** Pharmazeutische Zubereitungen enthaltend als Wirkstoff mindestens eine Verbindung der Formel (I) nach einem der Ansprüche 1 bis 13 allein oder in Kombination mit einem oder mehreren inerten, nichttoxischen, pharmazeutisch annehmbaren Trägermaterialien oder Bindemitteln.

**18.** Pharmazeutische Zubereitungen nach Anspruch 17 enthaltend mindestens einen Wirkstoff nach einem der Ansprüche 1 bis 13 zur Behandlung von Krebs.

## Claims

**1.** Compounds of formula (I) :

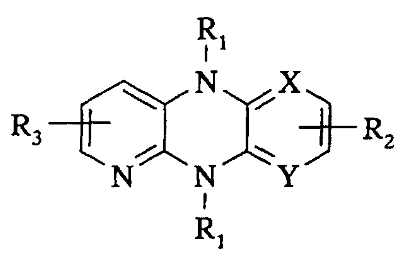

(I)

wherein:

**X** represents a nitrogen atom, a carbon atom, or a CH group,

**Y** represents a nitrogen atom when X represents a carbon atom or a CH group, or Y represents a carbon atom or a CH group when X represents a nitrogen atom,

with the proviso that when X and Y represent a carbon atom, that carbon atom carries the $R_2$ group,

**$R_1$** represents a linear or branched ($C_1$-$C_6$)alkyl group (optionally substituted by one or more identical or different groups selected from halogen, linear or branched ($C_1$-$C_6$)alkylthio, linear or branched ($C_1$-$C_6$)alkoxy, monoalkyl- or dialkyl-amino wherein alkyl is linear or branched and has from 1 to 6 carbon atoms, aryl, hydroxy, formyl, linear or branched ($C_1$-$C_6$)alkoxycarbonyl, and carboxy), it being understood that the two $R_1$ groups have either identical group definitions or different group definitions,

**$R_2$ and $R_3$,** which may be identical or different, each independently of the other represent a Z group,
<u>or</u>
one of the groups $R_2$ and $R_3$ represents a W group and the other of the groups $R_2$ and $R_3$ represents a Z group,
in which Z and W groups :

- Z represents :

  * a hydrogen atom,
  * a halogen atom,
  * a linear or branched ($C_1$-$C_6$)alkyl group, which alkyl group may be substituted by one or more identical or different groups selected from hydroxy, linear or branched ($C_1$-$C_6$)alkoxy, aryl, amino (optionally substituted by one or two identical or different groups selected from linear or branched ($C_1$-$C_6$)alkyl, aryl, aryl-($C_1$-$C_6$)alkyl in which alkyl is linear or branched, aminoalkyl in which alkyl is linear or branched and has from 1 to 6 carbon atoms, monoalkylaminoalkyl in which alkyl is linear or branched and has from 1 to 6 carbon atoms, dialkylaminoalkyl in which alkyl is linear or branched and has from 1 to 6 carbon atoms, linear or branched ($C_1$-$C_6$)-hydroxyalkyl, and alkoxyalkyl in which alkyl is linear or branched and has from 1 to 6 carbon atoms), and formyl,
  * amino (optionally substituted by one or two identical or different groups selected from linear or branched ($C_1$-$C_6$)alkyl, aryl, aryl-($C_1$-$C_6$)alkyl in which alkyl is linear or branched, linear or branched ($C_1$-$C_6$)alkylsulphonyl, arylsulphonyl, aminoalkyl in which alkyl is linear or branched and has from 1 to 6 carbon atoms, monoalkylaminoalkyl in which alkyl is linear or branched and has from 1 to 6 carbon atoms, dialkylaminoalkyl in which alkyl is linear or branched and has from 1 to 6 carbon atoms, linear or branched ($C_1$-$C_6$)hydroxyalkyl, and alkoxyalkyl in which alkyl is linear or branched and has from 1 to 6 carbon atoms),
  * nitro,
  * linear or branched ($C_1$-$C_6$)alkylthio,
  * formyl,
  * hydroxycarbonyl,
  * linear or branched ($C_1$-$C_6$)alkoxycarbonyl,
  * aminocarbonyl, monoalkylaminocarbonyl in which alkyl is linear or branched and has from 1 to 6 carbon atoms, or a dialkylaminocarbonyl group in which alkyl is linear or branched and has from 1 to 6 carbon atoms,

- W represents a linear or branched ($C_5$-$C_{24}$)alkylene chain, in which one or more carbon atoms is/are optionally replaced by one or more identical or different groups each independently of the other selected from oxygen atoms, imine groups, -N($R_4$)-CO-, -CO-N($R_4$)- and N($R_4$) groups wherein $R_4$ represents a hydrogen atom, a linear or branched ($C_1$-$C_6$)alkyl group, an aryl group, or an aryl-($C_1$-$C_6$)alkyl group in which alkyl is linear or branched, which alkylene chain is substituted in its terminal position by any one of the following groups:

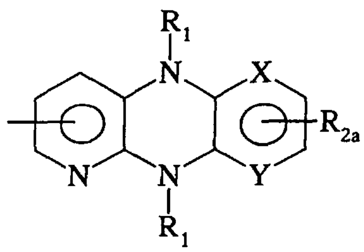,
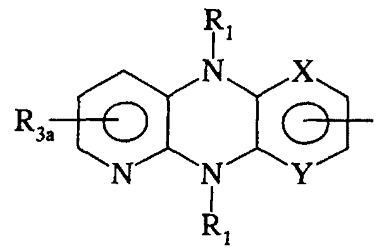

wherein X, Y and $R_1$ are as defined above, and $R_{2a}$ and $R_{3a}$ represent a Z group as defined above,

their isomers and addition salts thereof with a pharmaceutically acceptable acid or base, it being understood that "aryl group" is understood to mean a phenyl, naphthyl, tetrahydronaphthyl, dihydronaphthyl, indenyl or indanyl group, each of those groups optionally being substituted identically or differently by one or more : halogen atoms, hydroxy groups, cyano groups, nitro groups, linear or branched $(C_1-C_6)$alkyl groups, linear or branched trihalo-$(C_1-C_6)$alkyl groups, linear or branched $(C_1-C_6)$alkoxy groups, linear or branched $(C_1-C_6)$acyl groups, carboxy groups, linear or branched $(C_1-C_6)$alkoxycarbonyl groups, and amino groups (amino optionally being substituted by one or two identical or different groups selected from linear or branched $(C_1-C_6)$alkyl, aryl, and aryl-$(C_1-C_6)$ alkyl in which alkyl is linear or branched).

2. Compounds of formula (I) according to claim 1, **characterised in that** $R_2$ and $R_3$, which may be identical or different, each independently of the other represent a Z group, their isomers and addition salts thereof with a pharmaceutically acceptable acid or base.

3. Compounds of formula (I) according to claim 1, **characterised in that** one of the groups $R_2$ and $R_3$ represents a Z group and the other of the groups $R_2$ and $R_3$ represents a W group, their isomers and addition salts thereof with a pharmaceutically acceptable acid or base.

4. Compounds of formula (I) according to any one of claims 1 or 3, **characterised in that** one of the groups $R_2$ and $R_3$ represents a Z group and the other of the groups $R_2$ and $R_3$ represents a $W_1$ group, which $W_1$ group represents a linear $(C_6-C_{12})$alkylene chain in which two or three carbon atoms are replaced by two or three identical or different $N(R_4)$ groups wherein $R_4$ represents a hydrogen atom or a linear or branched $(C_1-C_6)$alkyl group, which alkylene chain is substituted in its terminal position by any one of the following two groups:

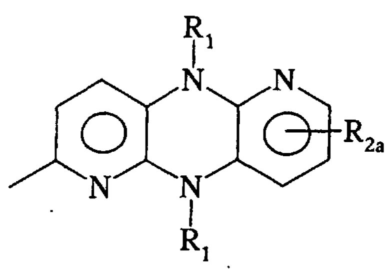,
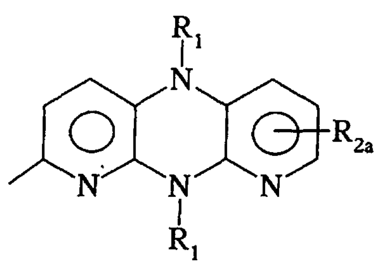

wherein $R_1$ is as defined for the general formula (I) and $R_{2a}$ represents a Z group, their isomers and addition salts thereof with a pharmaceutically acceptable acid or base.

5. Compounds of formula (I) according to any one of claims 1 or 3, **characterised in that** one of the groups $R_2$ and $R_3$ represents a Z group and the other of the groups $R_2$ and $R_3$ represents a $W_2$ group of formula:

$$-CO-N(R_4)-W'_2-N(R_4)-CO-G$$

wherein:

- each of the $R_4$ groups, which may be identical or different, represents a hydrogen atom or a linear or branched $(C_1-C_6)$alkyl group,
- $W'_2$ represents a linear $(C_3-C_{12})$alkylene chain in which one, two or three carbon atoms are optionally replaced by one, two or three identical or different $N(R_4)$ groups wherein $R_4$ represents a hydrogen atom or a linear or branched $(C_1-C_6)$alkyl group, and
- G represents any one of the following two groups:

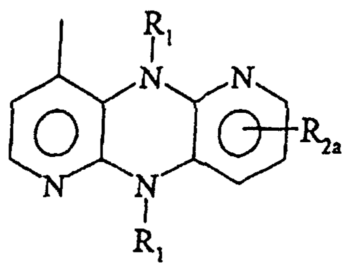 , 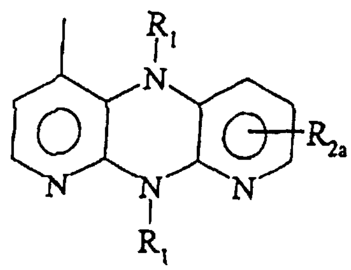

wherein $R_1$ is as defined for the general formula (I) and $R_{2a}$ represents a Z group, their isomers and addition salts thereof with a pharmaceutically acceptable acid or base.

**6.** Compounds of formula (I) according to claim 4, **characterised in that**:

- one of the groups $R_2$ and $R_3$ represents a $W_1$ group wherein the $R_1$ group represents a linear or branched $(C_1-C_6)$alkyl group, and the group $R_{2a}$ represents a hydrogen atom,
- the other of the groups $R_2$ and $R_3$ represents a Z group corresponding to a hydrogen atom, their isomers and addition salts thereof with a pharmaceutically acceptable acid or base.

**7.** Compounds of formula (I) according to claim 5, **characterised in that**:

- one of the groups $R_2$ and $R_3$ represents a $W_2$ group wherein the $R_1$ group represents a linear or branched $(C_1-C_6)$alkyl group, and the $R_{2a}$ group represents a hydrogen atom,
- the other of the groups $R_2$ and $R_3$ represents a Z group corresponding to a hydrogen atom, their isomers and addition salts thereof with a pharmaceutically acceptable acid or base.

**8.** Compounds of formula (I) according to claim 1, **characterised in that** $R_1$ represents a linear or branched $(C_1-C_6)$ alkyl group, their isomers and addition salts thereof with a pharmaceutically acceptable acid or base.

**9.** Compound of formula (I) according to claim 1 which is N1,N2-di[(5,10-dimethyl-5,10-dihydrodipyrido[2,3-*b* : 3,2-*e*]pyrazin-2-yl)methyl]-1,2-ethanediamine, its isomers and addition salts thereof with a pharmaceutically acceptable acid or base.

**10.** Compound of formula (I) according to claim 1 which is N1,N3-di[(5,10-dimethyl-5,10-dihydrodipyrido[2,3-*b*: 3,2-*e*]pyrazin-2-yl)methyl]-1,3-propanediamine, its isomers and addition salts thereof with a pharmaceutically acceptable acid or base.

**11.** Compound of formula (I) according to claim 1 which is N1,N4-di[(5,10-dimethyl)-5,10-dihydrodipyrido[2,3-*b* : 3,2-*c*]pyrazin-2-yl)methyl]-1,4-butanediamine, its isomers and addition salts thereof with a pharmaceutically acceptable acid or base.

**12.** Compound of formula (I) according to claim 1 which is N1-[(5,10-dimethyl-5,10-dihydrodipyrido-[2,3-*b* : 3,2-*e*]pyrazin-2-yl)methyl]-N3-(3-{[5,10-dimethyl-5,10-dihydrodipyrido[2,3-*b* : 3,2-*e*]pyrazin-2-yl)methyl]amino}propyl)-N3-methyl-1,3-propanediamine, its isomers and addition salts thereof with a pharmaceutically acceptable acid or base.

**13.** Compound of formula (I) according to claim 1 which is N1,N7-di[(5,10-dimethyl-5,10-dihydrodipyrido-[2,3-*b* : 3,2-*e*]pyrazin-2-yl)methyl]-1,7-heptanediamine, its isomers and addition salts thereof with a pharmaceutically acceptable acid or base

**14.** Process for the preparation of the compounds of formula (I) according to claim 1, **characterised in that** there is used as starting material a 3-halo-2-amino-pyridine of formula (II) :

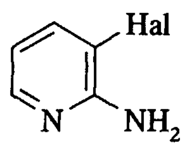

**(II)**

wherein Hal represents a halogen atom,
which compounds of formula (II) are reacted under basic conditions with a compound of formula (**III**) :

$R'_1$ - Hal **(III)**

wherein Hal represents a halogen atom and $R'_1$ represents a linear or branched ($C_1$-$C_6$)alkyl group, optionally substituted by a linear or branched ($C_1$-$C_6$)alkoxy group, a monoalkyl- or dialkyl-amino group wherein alkyl is linear or branched and has from 1 to 6 carbon atoms, or by an aryl group,
to yield the compounds of formula **(IV)** :

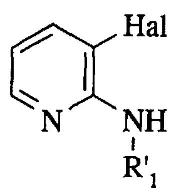

**(IV)**

wherein Hal and $R'_1$ are as defined above,
which compound of formula (IV) is condensed with itself in the presence of a complex base, to yield the compounds of formula **(I/a)**, a particular case of the compounds of formula (I) :

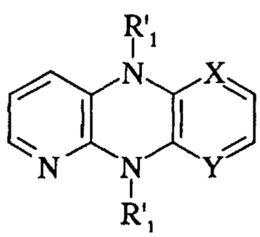

**(I/a)**

wherein $R'_1$ is as defined above and X and Y are as defined for formula (I),
which compounds of formula (I/a), in the particular case where $R'_1$ represents an $R''_1$ group consisting of a linear ($C_1$-$C_6$)alkyl group, are subjected, if desired, to a metallation reaction and the intermediate anion of which is trapped by a ($C_1$-$C_6$)alkyl halide, a di($C_1$-$C_6$)alkyl disulphide, alkyl being linear or branched, a linear or branched ($C_1$-$C_6$) alkyl-($C_1$-$C_6$)-alkoxy halide, a halogen atom, an aldehyde, a ketone, a carboxyl compound or a linear or branched ($C_1$-$C_6$)alkoxycarbonyl compound, to yield the compounds of formulae **(I/b)** and **(I/b')**, and/or **(I/c)**, the products (I/b') being co-reaction products :

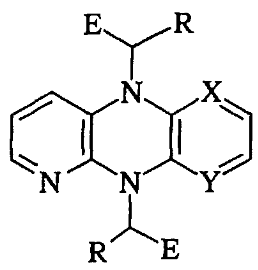
(and/or)
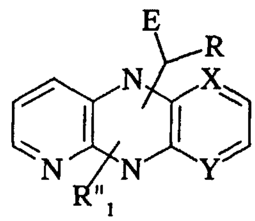
,
(and/or)
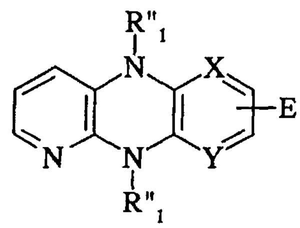

**(I/b)** **(I/b')** **(I/c)**

wherein X, Y and $R''_1$ are as defined above, R represents a hydrogen atom or a linear $(C_1-C_5)$alkyl group and E represents a halogen atom, a linear or branched $(C_1-C_6)$alkyl group, a linear or branched $(C_1-C_6)$alkylthio group, a formyl group, a linear or branched $(C_1-C_6)$alkyl group substituted by a linear or branched $(C_1-C_6)$alkoxy group, or a linear or branched $(C_1-C_6)$alkyl group substituted by one or more identical or different groups selected from aryl, hydroxy, formyl, linear or branched $(C_1-C_6)$alkoxycarbonyl, and carboxy,
the totality of the compounds of formulae (I/a), (I/b) and (I/b') constituting the compounds of formula **(I/d)** :

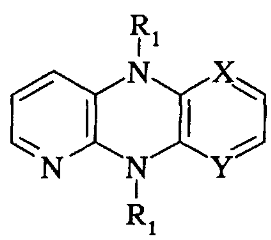
**(I/d)**

wherein $R_1$, X and Y are as defined for formula (I),
which compounds of formula (I/d),

- are treated, if desired, with a nitrating agent, to yield the compounds of formula **(I/e)**, a particular case of the compounds of formula (I),

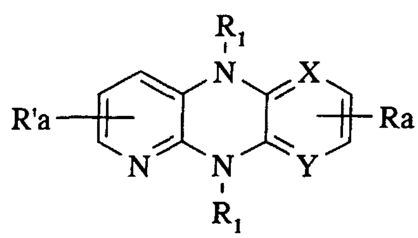
**(I/e)**

wherein X, Y and $R_1$ are as defined above,
and Ra and R'a, which may be identical or different, represent a hydrogen atom or a nitro group, it being understood that Ra and R'a cannot simultaneously represent a hydrogen atom,
the nitro function(s) of which compounds of formula (I/e) is/are, if desired, reduced to the amine function, to yield the compounds of formula **(I/f)**, a particular case of the compounds of formula (I):

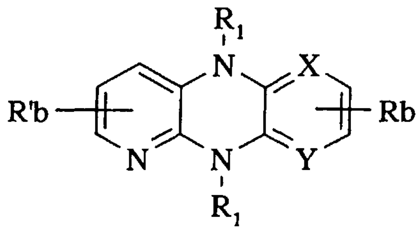

**(I/f)**

wherein X, Y and $R_1$ are as defined above and Rb and R'b, which may be identical or different, represent a hydrogen atom or an amino group, it being understood that Rb and R'b cannot simultaneously represent a hydrogen atom, which compounds of formula (I/f) are treated under basic conditions with an electrophilic compound, to yield the compounds of formula **(I/g)**, a particular case of the compounds of formula (I) :

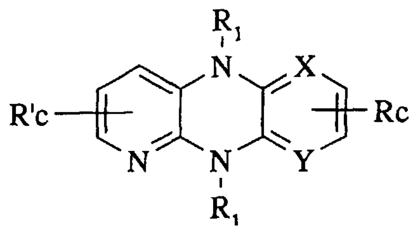

**(I/g)**

wherein X, Y and $R_1$ are as defined above, and Rc and R'c, which may be identical or different, represent a hydrogen atom or an amino group substituted by one or two identical or different groups selected from linear or branched $(C_1\text{-}C_6)$alkyl, aryl, aryl-$(C_1\text{-}C_6)$alkyl in which alkyl is linear or branched, linear or branched $(C_1\text{-}C_6)$ alkylsulphonyl, arylsulphonyl, aminoalkyl in which alkyl is linear or branched and has from 1 to 6 carbon atoms, monoalkylaminoalkyl in which alkyl is linear or branched and has from 1 to 6 carbon atoms, dialkylaminoalkyl in which alkyl is linear or branched and has from 1 to 6 carbon atoms, linear or branched $(C_1\text{-}C_6)$hydroxyalkyl, and alkoxyalkyl in which alkyl is linear or branched and has from 1 to 6 carbon atoms, it being understood that Rc and R'c cannot simultaneously represent a hydrogen atom,

❖ or which compounds of formula (I/d) are subjected to conditions of a formylation reaction, to yield the compounds of formula **(I/h)**, a particular case of the compounds of formula (I) :

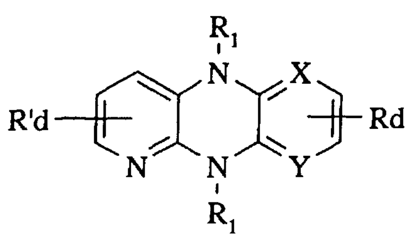

**(I/h)**

wherein X, Y and $R_1$ are as defined above and Rd and R'd, which may be identical or different, represent a formyl group or a hydrogen atom, it being understood that Rd and R'd cannot simultaneously represent a hydrogen atom,
which compounds of formula (I/h)

- are treated with an oxidising agent and are then optionally subjected to the action of an alcohol, to yield the compounds of formula **(I/h')**, a particular case of the compounds of formula (I) :

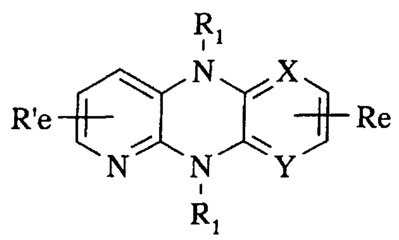 **(I/h')**

wherein $R_1$, X and Y are as defined above and Re and R'e, which may be identical or different, represent a hydrogen atom, a hydroxycarbonyl group or a linear or branched ($C_1$-$C_6$)alkoxycarbonyl group, it being understood that R'e and Re cannot simultaneously represent a hydrogen atom,
in which compounds of formula (I/h') optionally at least one of the groups Re and/or R'e is converted to the acid chloride, according to conventional conditions of organic synthesis, or which compounds are reacted directly with an amino compound, for example according to conditions of peptide coupling, to yield the compounds of formula (I/h'), a particular case of the compounds of formula (I):

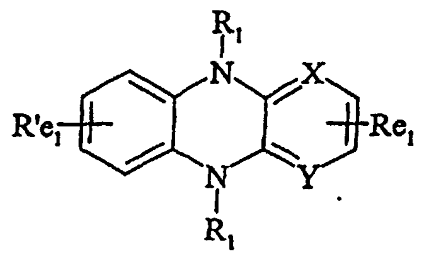 **(I/h')**

wherein $R_1$, X and Y are as defined above and $Re_1$ and $R'e_1$, which may be identical or different, represent a hydrogen atom, an aminocarbonyl group, a monoalkylaminocarbonyl group in which alkyl is linear or branched and has from 1 to 6 carbon atoms, or dialkylaminocarbonyl in which alkyl is linear or branched and has from 1 to 6 carbon atoms, it being understood that $Re_1$ and $R'e_1$ cannot simultaneously represent a hydrogen atom,

- or are subjected to conditions of reductive amination to yield the compounds of formula **(I/i)**, a particular case of the compounds of formula (I) :

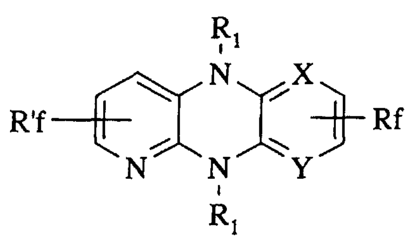 **(I/i)**

wherein X, Y and $R_1$ are as defined above and Rf and R'f, which may be identical or different, represent a hydrogen atom or a group of formula $CH_2$-$NR_{10}$-$R_{20}$ wherein $R_{10}$ and $R_{20}$, which may be identical or different, represent a hydrogen atom, a linear or branched ($C_1$-$C_6$)alkyl group, an aryl group, an aryl-($C_1$-$C_6$)alkyl group in which alkyl is linear or branched, an aminoalkyl group in which alkyl is linear or branched and has from 1 to 6 carbon atoms, a monoalkylaminoalkyl group in which alkyl is linear or branched and has from 1 to 6 carbon atoms, a dialkylaminoalkyl group in which alkyl is linear or branched and has from 1 to 6 carbon atoms, a linear or branched ($C_1$-$C_6$)hydroxyalkyl group, or an alkoxyalkyl group in which alkyl is linear or branched and has from 1 to 6 carbon atoms, it being understood that Rf and R'f cannot simultaneously represent a hydrogen atom,

- or are treated with a phosphorus ylid prepared starting from a compound of formula **(V)**:

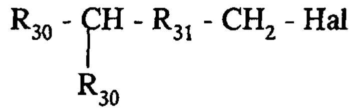 **(V)**

wherein Hal represents a halogen atom, $R_{31}$ represents a linear or branched ($C_1$-$C_4$)alkylene chain, and $R_{30}$ represents an aldehyde-protecting group, such as an acetal,
to yield the compounds of formula **(VI)** :

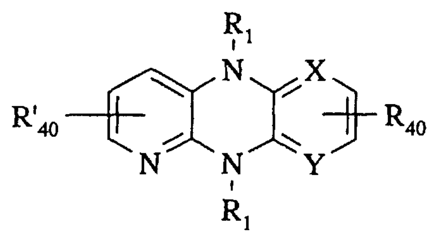 **(VI)**

wherein X, Y and $R_1$ are as defined above and $R_{40}$ and $R'_{40}$ represent a hydrogen atom or a group of formula:

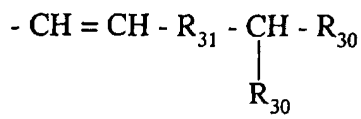

wherein $R_{31}$ and $R_{30}$ are as defined above,
the double bond of which compounds of formula (VI) is reduced according to conventional conditions of organic synthesis, and the terminal aldehyde function of which is then deprotected, to yield the compounds of formula **(I/j)**, a particular case of the compounds of formula (I) :

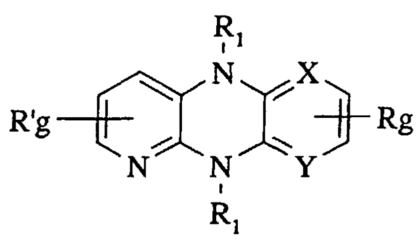 **(I/j)**

wherein X, Y and $R_1$ are as defined above and Rg and R'g, which may be identical or different, represent a hydrogen atom or a linear or branched ($C_1$-$C_6$)alkyl group, substituted by a formyl group, it being understood that Rg and R'g cannot simultaneously represent a hydrogen atom,
which compounds of formula (I/j) are subjected to conditions of reductive amination to yield the compounds of formula **(I/k)**, a particular case of the compounds of formula (I):

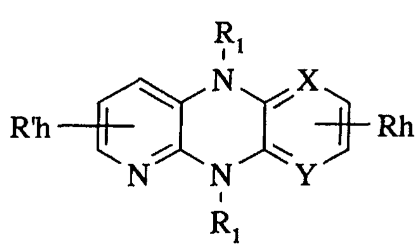 **(I/k)**

wherein X, Y and $R_1$ are as defined above and Rh and R'h, which may be identical or different, represent a hydrogen atom or a linear or branched ($C_1$-$C_6$)alkyl group, substituted by a group of formula $CH_2$-$NR_{10}R_{20}$ wherein $R_{10}$ and $R_{20}$ are as defined above, it being understood that Rh and R'h cannot simultaneously represent a hydrogen atom,

❖ or which compounds of formula (I/d) are subjected to the action of a halogenating agent, such as, for example, phenyltrimethylammonium tribromide, to yield the compounds of formula **(I/l)**, a particular case of the compounds of formula (I) :

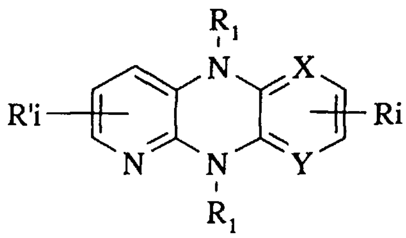

**(I/l)**

wherein X, Y and $R_1$ are as defined above and Ri and R'i, which may be identical or different, represent a hydrogen atom or a halogen atom, it being understood that Ri and R'i do not simultaneously represent a hydrogen atom,
it being understood that the reactions described above, carried out starting from the compounds of formula (I/d), enable there to be obtained one or more products substituted on one pyridyl ring or on the other pyridyl ring, or on both pyridyl rings, that those various reactions may be carried out in any order starting from the compounds of formula (I/d) and/or (I/c), and that, by judicious selection on the part of the person skilled in the art, in terms of the reagents used, the order in which the reactions are carried out, and the reaction conditions, it is possible to obtain, starting from the compounds of formulae (I/d) and (I/c), any one of the compounds of formula **(I/m)**, a particular case of the compounds of formula (I) :

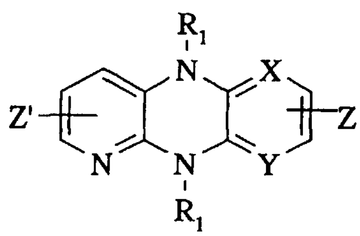

**(I/m)**

wherein X, Y, $R_1$ and Z are as defined for formula (I), and Z' represents any of the definitions of Z, *which compounds of formula (I/m)* are treated, if desired,

⇨ when one of the groups Z and Z' represents an amino group or an aminoalkyl group in which alkyl is linear or branched and has from 1 to 7 carbon atoms, under conditions of amination or reductive amination,

* *with a compound* of formula **(VII)** :

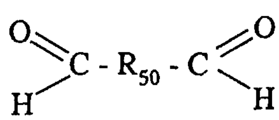

**(VII)**

wherein $R_{50}$ represents a linear or branched alkylene chain, wherein one or more carbon atoms is/are optionally replaced by one or more oxygen atoms or $N(R_4)$ groups wherein $R_4$ is as defined for formula (I),

* or *with a compound* of formula **(VIII)** :

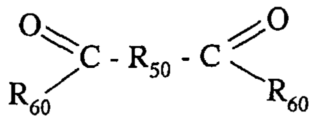

**(VIII)**

wherein $R_{60}$ represents a chlorine atom or a linear or branched ($C_1$-$C_6$)alkoxy group, and $R_{50}$ is as defined above,

⇨ or, when one of the groups Z and Z' represents a formyl group or a linear or branched formyl-($C_1$-$C_6$)alkyl group, under conditions of amination or reductive animation, with a compound of formula **(IX)** :

$$HR_4N - R_{70} - NR_4H \qquad \textbf{(IX)}$$

wherein $R_4$ is as defined for formula (I) and $R_{70}$ represents a linear or branched alkylene chain wherein one or more carbon atoms may optionally be replaced by one or more oxygen atoms or $NR_4$ groups wherein $R_4$ is as defined above,

⇨ or, when one of the groups Z and Z' represents a linear or branched ($C_1$-$C_6$)-alkoxycarbonyl group, or an acid chloride, with a compound of formula (IX) as defined above, under conventional conditions of peptide coupling,
to yield the compounds of formula **(I/n)** or **(I/o)**, a particular case of the compounds of formula (I) :

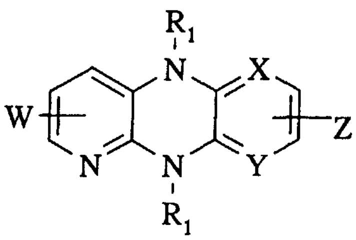

**(I/n)**

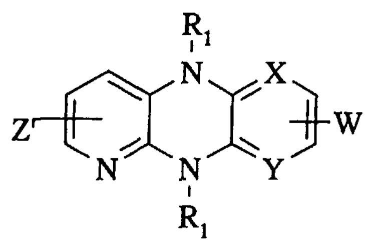

**(I/o)**

wherein X, Y, $R_1$, Z and W are as defined for formula (I), and Z' is as defined above, it being understood that the chain length of the groups $R_{50}$ and $R_{70}$ as defined above is such that it is possible to obtain compounds (I/n) and (I/o) wherein W has from 5 to 24 chain members,
which compounds (I/a) to (I/o) constitute the totality of the compounds of the invention, which are purified, if necessary, according to a conventional purification technique, which may, if desired, be separated into their different isomers according to a conventional separation technique, and which are converted, where appropriate, into addition salts thereof with a pharmaceutically acceptable acid or base.

**15.** Compounds of formulae (α), (β) and (χ) :

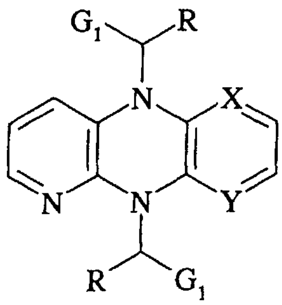
,
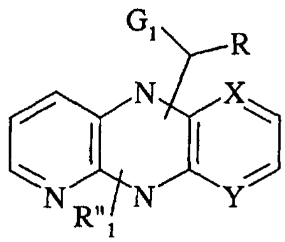
,

(α) (β)

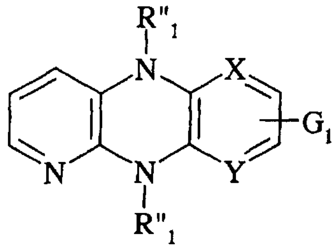

(χ)

wherein:

X and Y are as defined for formula (I),
R"$_1$ represents a linear ($C_1$-$C_6$)alkyl group,
R represents a hydrogen atom or a linear ($C_1$-$C_5$)alkyl group,
$G_1$ represents a tin, boron or silicon atom, each of those atoms being substituted by one or more linear or branched ($C_1$-$C_6$)alkyl groups,
for use as intermediates in the synthesis of the compounds of formula (I).

**16.** Process for the preparation of the compounds of formulae (α) and (β) and/or (χ), for use as intermediates in the synthesis of the compounds of formula (I), **characterised in that** a compound of formula (I/a), a particular case of the compounds of formula (I) :

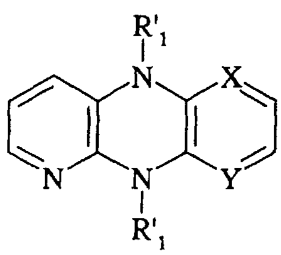

**(I/a)**

wherein X and Y are as defined for formula (I) and R'$_1$ represents an R"$_1$ group consisting of a linear ($C_1$-$C_6$)alkyl group,
is subjected to a metallation reaction in which the intermediate anion is trapped by a tin, boron or silicon compound, enabling the compounds of formulae (α) and (β) and/or (χ) to be obtained:

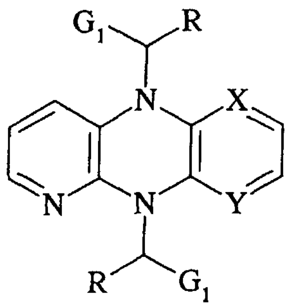

(α)

(and/or)

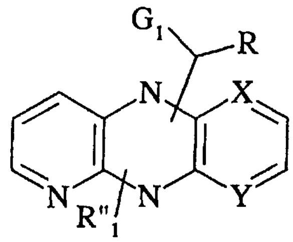

(β)

(and/or)

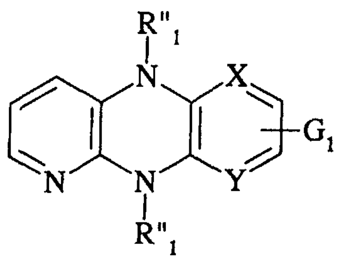

(χ)

wherein:

X, Y and $R''_1$ are as defined above, R represents a hydrogen atom or a linear ($C_1$-$C_5$)alkyl group, and $G_1$ represents a tin, boron or silicon atom, each of those atoms being substituted by one or more linear or branched ($C_1$-$C_6$)alkyl groups.

**17.** Pharmaceutical compositions comprising as active ingredient at least one compound of formula (I) according to any one of claims 1 to 13, on its own or in combination with one or more inert, non-toxic pharmaceutically acceptable excipients or carriers.

**18.** Pharmaceutical compositions according to claim 17 comprising at least one active ingredient according to any one of claims 1 to 13, for use in the treatment of cancer.